# EUROPEAN PATENT APPLICATION

(11) **EP 3 670 527 A1**
(43) Date of publication of application: **24.06.2020**
(21) Application number: 18215370.0
(22) Date of filing: 21.12.2018
(51) Int. Cl.: C07K 14/375, C12N 15/82

(54) **AGERITIN AS BIOINSECTICIDE AND METHODS OF GENERATING AND USING IT**

(71) Applicant: Senckenberg Gesellschaft Für Naturforschung, 60325 Frankfurt Am Main (DE); ETH Zurich, 8092 Zürich (CH)
(72) Inventor: HENNICKE, Florian, 60488 Frankfurt am Main (DE); KÜNZLER, Markus, 5420 Ehrendingen (CH); TAYYROV, Annageldi, 8057 Zürich (CH); LÜTHY, Peter, 8706 Feldmeilen (CH)
(74) Representative: Grahn, Sibylla Maria

(57) **Abstract**

The present invention relates to the fungal protein ageritin, a nucleic acid molecule encoding said protein, host cells expressing the protein and/or the nucleic acid molecule and a plant or fungus expressing the protein and/or the nucleic acid molecule and/or comprising such host cells. The present invention further relates to using the fungal protein ageritin, the nucleic acid molecule encoding it, the host cell expressing it and/or the plant as bioinsecticide(s). The present invention further relates to a bioinsecticide composition.

SEQUENCE LISTING

<110>  Senckenberg Gesellschaft für Naturforschung
       ETH Zurich

<120>  Ageritin as bioinsecticide and methods of generating and using it

<130>  S33603EP

<160>  32

<170>  PatentIn version 3.5

<210>  1
<211>  156
<212>  PRT
<213>  Agrocybe aegerita

<400>  1

Met Ser Glu Ser Ser Thr Phe Thr Thr Ala Val Val Pro Glu Gly Glu
1               5                   10                  15

Gly Val Ala Pro Met Ala Glu Thr Val Gln Tyr Tyr Asn Ser Tyr Ser
            20                  25                  30

Asp Ala Ser Ile Ala Ser Cys Ala Phe Val Asp Ser Gly Lys Asp Lys
        35                  40                  45

Ile Asp Lys Thr Lys Leu Val Thr Tyr Thr Ser Arg Leu Ala Ala Ser
    50                  55                  60

Pro Ala Tyr Gln Lys Val Val Gly Val Gly Leu Lys Thr Ala Ala Gly
65                  70                  75                  80

Ser Ile Val Pro Tyr Val Arg Leu Asp Met Asp Asn Thr Gly Lys Gly
                85                  90                  95

Ile His Phe Asn Ala Thr Lys Leu Ser Asp Ser Ser Ala Lys Leu Ala
            100                 105                 110

Ala Val Leu Lys Thr Thr Val Ser Met Thr Glu Ala Gln Arg Thr Gln
        115                 120                 125

Leu Tyr Met Glu Tyr Ile Lys Gly Ile Glu Asn Arg Ser Ala Gln Phe
    130                 135                 140

Ile Trp Asp Trp Trp Arg Thr Gly Lys Ala Pro Ala
145                 150                 155

<210>  2
<211>  471
<212>  DNA

<213>  Agrocybe aegerita

<400>  2
atgtccgagt cctctacctt caccactgcg gtagtacctg aaggcgaagg agttgctcca      60

atggcagaga ccgtgcagta ttacaactcc tactctgacg catccatcgc gtcttgcgca     120

tttgtagact cggggaagga caaaattgat aagaccaagt tggtcacgta caccagccgc     180

ctcgccgcaa gccccgcata tcagaaggtc gtcggcgtcg gcctcaaaac ggccgcgggc     240

tccatcgtgc cctacgtccg gctcgacatg gacaacaccg gcaagggcat ccatttcaac     300

gcgactaaac tctccgacag ttccgccaag ctcgccgcgg tgctcaagac gacggtgtcc     360

atgaccgagg cacagcgaac tcaactctac atggagtata tcaagggcat cgagaatcgg     420

agtgcgcagt ttatttggga ctggtggagg acgggcaagg ctccggcgtg a              471


<210>  3
<211>  151
<212>  PRT
<213>  Agrocybe aegerita

<400>  3

Met Ser Asp Pro Ser Ala Pro Gly Leu Glu Glu Gly Thr Glu Ile Ser
1               5                   10                  15


Pro Met Ala Glu Thr Val Gln Thr Phe Ala Ser Tyr Ser Glu Ala Ser
            20                  25                  30


Val Ala Ala Cys Lys Trp Val Asn Ser Gly Lys Thr Gln Ile Asp Pro
        35                  40                  45


Ala Gln Leu Ile Leu Tyr Lys Asn Thr Leu Pro Ala Ser Pro Ala Tyr
    50                  55                  60


Gly Lys Ile Val Gly Val Gly Leu Lys Phe Thr Ala Glu Val Asp Phe
65                  70                  75                  80


Cys Arg Leu Asp Met Asp Asn Thr Gly Lys Gly Ile His Phe Asn Ala
                85                  90                  95


Lys Gln Arg Asp Asp Gln Ser Lys Lys Leu Ala Ala Val Ile Lys Pro
            100                 105                 110


Thr Val Ala Leu Ser Glu Ala Gln Arg Thr Gln Leu Tyr Met Glu Tyr
        115                 120                 125


Ile Lys Gly Leu Glu Asn Arg Ser Ala Gln Phe Ile Trp Glu Trp Trp
    130                 135                 140

Ser Thr Gly Lys Ala Pro Ala
145                 150

<210>  4
<211>  456
<212>  DNA
<213>  Agrocybe aegerita

<400>  4
atgtctgacc catccgcacc cggactcgaa gagggcaccg aaatctcacc catggccgag      60

acggtgcaga ccttcgcgag ctactccgag gcctccgttg ccgcttgcaa gtgggtcaac     120

tcgggaaaga cccagattga cccagcccag ctcatcctgt acaagaacac cctccccgca     180

agccccgcat acggcaaaat cgtcggcgtc ggcctaaaat tcaccgccga ggtcgacttc     240

tgccgactcg acatggacaa cacaggcaag ggcatccact tcaacgccaa gcagcgggac     300

gaccagtcca agaagctcgc ggcggtgatc aagccgactg ttgcgttgag cgaggcccag     360

cgcacgcagc tgtacatgga gtatatcaag gggctcgaga acaggagtgc gcagtttatt     420

tgggagtggt ggagcaccgg gaaggcaccg gcgtga                               456

<210>  5
<211>  36
<212>  DNA
<213>  Artificial Sequence

<220>
<223>  Primer

<400>  5
ggcgcatatg tccgagtcct ctaccttcac cactgc                                36

<210>  6
<211>  29
<212>  DNA
<213>  Artificial Sequence

<220>
<223>  Primer

<400>  6
gtgcggccgc tcacgccgga gccttgccc                                        29

<210>  7
<211>  34
<212>  DNA
<213>  Artificial Sequence

<220>
<223>  Primer

<400>  7
caccaccacc acgagtcctc taccttcacc actg                                  34

<210>  8
<211>  34
<212>  DNA
<213>  Artificial Sequence

<220>
<223>  Primer

<400>  8
atgatgatga tgggacatat gtatatctcc ttct                                    34


<210>  9
<211>  25
<212>  DNA
<213>  Artificial Sequence

<220>
<223>  Primer

<400>  9
aagttggtca cggccaccag ccgcc                                              25


<210>  10
<211>  26
<212>  DNA
<213>  Artificial Sequence

<220>
<223>  Primer

<400>  10
ggtcttatca attttgtcct tccccg                                             26


<210>  11
<211>  24
<212>  DNA
<213>  Artificial Sequence

<220>
<223>  Primer

<400>  11
gtcgcgctcg acatggacaa cacc                                               24


<210>  12
<211>  24
<212>  DNA
<213>  Artificial Sequence

<220>
<223>  Primer

<400>  12
gtagggcacg atggagcccg cggc                                               24


<210>  13
<211>  24
<212>  DNA

<213>  Artificial Sequence

<220>
<223>  Primer

<400>  13
tgccctacgt ccggctcgcc atgg                                              24


<210>  14
<211>  24
<212>  DNA
<213>  Artificial Sequence

<220>
<223>  Primer

<400>  14
cgatggagcc cgcggccgtt ttga                                              24


<210>  15
<211>  24
<212>  DNA
<213>  Artificial Sequence

<220>
<223>  Primer

<400>  15
gtccggctcg acatggccaa cacc                                              24


<210>  16
<211>  24
<212>  DNA
<213>  Artificial Sequence

<220>
<223>  Primer

<400>  16
gtagggcacg atggagcccg cggc                                              24


<210>  17
<211>  20
<212>  DNA
<213>  Artificial Sequence

<220>
<223>  Primer

<400>  17
ggcaagggca tcgctttcaa                                                   20


<210>  18
<211>  20
<212>  DNA
<213>  Artificial Sequence

<220>

<223>  Primer

<400>  18
ggtgttgtcc atgtcgagcc                                                   20


<210>  19
<211>  19
<212>  DNA
<213>  Artificial Sequence

<220>
<223>  Primer

<400>  19
agttccgccg cgctcgccg                                                    19


<210>  20
<211>  25
<212>  DNA
<213>  Artificial Sequence

<220>
<223>  Primer

<400>  20
gtcggagagt ttagtcgcgt tgaaa                                             25


<210>  21
<211>  25
<212>  DNA
<213>  Artificial Sequence

<220>
<223>  Primer

<400>  21
ttcttttcgc tactcagaat cgttg                                             25


<210>  22
<211>  20
<212>  DNA
<213>  Artificial Sequence

<220>
<223>  Primer

<400>  22
cagagctctc ccaaccacag                                                   20


<210>  23
<211>  24
<212>  DNA
<213>  Artificial Sequence

<220>
<223>  Primer

<400>  23

agatgcgtat tctgatggtt ggtc                                                24

<210>  24
<211>  23
<212>  DNA
<213>  Artificial Sequence

<220>
<223>  Primer

<400>  24
cccacactgt gaatgagatg ttc                                                 23

<210>  25
<211>  21
<212>  DNA
<213>  Artificial Sequence

<220>
<223>  Primer

<400>  25
attcctacga tccttttgcc g                                                   21

<210>  26
<211>  23
<212>  DNA
<213>  Artificial Sequence

<220>
<223>  Primer

<400>  26
gatcatattg tttcgggagt cct                                                 23

<210>  27
<211>  21
<212>  DNA
<213>  Artificial Sequence

<220>
<223>  Primer

<400>  27
tctgatctga ctgtcggcca a                                                   21

<210>  28
<211>  21
<212>  DNA
<213>  Artificial Sequence

<220>
<223>  Primer

<400>  28
atcctcgtcc ttatgctcct c                                                   21

<210>  29
<211>  19
<212>  DNA
<213>  Artificial Sequence

<220>
<223>  Primer

<400>  29
aagccccgca tatcagaag                                                    19


<210>  30
<211>  20
<212>  DNA
<213>  Artificial Sequence

<220>
<223>  Primer

<400>  30
ctgtcggaga gtttagtcgc                                                   20


<210>  31
<211>  21
<212>  DNA
<213>  Artificial Sequence

<220>
<223>  Primer

<400>  31
gaaagaccca gattgaccca g                                                 21


<210>  32
<211>  19
<212>  DNA
<213>  Artificial Sequence

<220>
<223>  Primer

<400>  32
gtgaatttta ggccgacgc                                                    19

## Description

The present invention relates to the fungal protein ageritin, a nucleic acid molecule encoding said protein, host cells expressing the protein and/or the nucleic acid molecule and a plant or fungus expressing the protein and/or the nucleic acid molecule and/or comprising such host cells. The present invention further relates to using the fungal protein ageritin, the nucleic acid molecule encoding it, the host cell expressing it and/or the plant as bioinsecticide(s). The present invention further relates to a bioinsecticide composition.

### BACKGROUND OF THE INVENTION

Fungi produce a variety of defense proteins against antagonists. One type of defense proteins are toxic ribonucleases also called ribotoxins. These toxins cleave a single phosphodiester bond within the universally conserved sarcin-ricin loop (SRL) of ribosomes and consequently inhibit protein synthesis.

Fungi are exposed to a large diversity of antagonists in their environment. To protect themselves, fungi mainly rely on (bio)chemical defense mediated by secondary metabolites, peptides and proteins (see e.g. Lacadena *et al.,* 2007). They interfere with essential biological processes or structures within the target organisms. Ribosomes are essential molecular machineries present in all living cells making them ideal targets for defense proteins. Previous studies have revealed three main classes of proteins with ribonucleolytic activity towards ribosomal RNA. The first class comprises classical ribonucleases (RNases) that cleave any phosphodiester bond between ribonucleotides. These RNases have a rather low specificity for ribosomal RNAs and include non-toxic RNases. The second class of proteins is represented by ribosome inactivating proteins (RIPs) that act on ribosomes. RIPs are *N*-glycosidases that depurinate a specific adenine residue located in the highly conserved sarcin-ricin loop (SRL) of the large subunit of the eukaryotic and prokaryotic ribosomes. The depurination of the adenine disrupts the binding of the translation elongation factors, inhibiting the protein synthesis and leading to the death of the cells. High effectiveness and specificity makes RIPs a part of the defense systems of many organisms including bacteria (Endo *et al.,* 1988), algae (Sawa *et al*., 2016), fungi (Yao *et al.,* 1998) and plants (Bolognesi *et al.,* 2016).

The third class of proteins with ribonucleolytic activity towards rRNA are called ribotoxins. They are small sized (10-20 kDa) and highly toxic (Lacadena *et al.,* 2007; Herrero-Galan *et al.,* 2009; Olombrada *et al.,* 2013). Ribotoxins are highly specific fungal endonucleases that cleave a single phosphodiester bond at a universally conserved GAGA tetraloop of the SRL loop. Similar to RIPs, the damage of the loop inhibits binding of translation elongation factors and thus protein biosynthesis, ultimately leading to the death of the target cells (Correll *et al*., 1998). Until recently, fungal ribotoxins were exclusively known from members of the phylum Ascomycota.Two of the best-studied ribotoxins are α-sarcin (Perez-Canadillas *et al*., 2000) and restrictocin (Yang *et al.,* 1996) produced by *Aspergillus giganteus* and *Aspergillus restrictus*, respectively. Hirsutellin A (Herrero-Galan *et al.,* 2008) from the fungal pathogen of mites, *Hirsutella thompsonii*, and anisoplin (Olombrada *et al.,* 2017) from the entomopathogenic fungus *Metarhizium anisopliae* are other, recently discovered ribotoxins. Lately, Landi *et al.* (2017) purified a protein, named ageritin, with ribonucleolytic activity towards rRNA from the commercially produced edible mushroom *Agrocybe aegerita*, a member of the phylum Basidiomycota. In addition to the RNase activity, the authors demonstrated cytotoxicity and cell death promoting effects of ageritin against tumor cell lines of the human central nervous system (CNS) tumor cell lines.

Tropical mosquito species like the yellow fever mosquito *Aedes aegypti* are important disease vectors for some of the most important infectious diseases globally, including the severe arboviral diseases such as yellow fever, chikungunya, dengue or zika.. Stopping the development from larvae into adult mosquitos by larviciding (killing of their larvae) can efficiently abolish the establishment and spreading of stable populations by such mosquitos and can hence minimize the risk of disease transmission. Larviciding is currently done by introducing the biopesticide *Bacillus thuringiensis israelensis* (Bti) into bodies of standing water as potential sites of egg laying and larval hatching. However, this practice is potentially at risk of becoming ineffective as different genetic resistance mechanisms by *Ae. aegypti* and other mosquitos such as the pestiferous *Culex quinquefasciatus* have been reported (Suter *et al.,* 2017).

However, this practice is potentially at risk of becoming ineffective as different reports on *Bti* resistance development by *Ae. aegypti* and other mosquitos such as the pestiferous *Culex quinquefasciatus* have been published (see e.g. Suter *et al.* 2017). In addition, the increasing spread of invasive *Aedes* mosquitoes leads to emergence of arboviral diseases such as dengue and chikungunya in more temperate regions (Barzon, 2018). For example, *Ae. albopictus* has invaded large parts of southern Europe. This insect is an efficient vector of dengue, chikungunya and zika (Kampen *et al.,* 2017).

Also, from a broader perspective, insecticide resistance to currently available insecticides is a key challenge. Protective interventions such as insecticide-treated bed nets and indoor residual spraying become more and more ineffective.

In conclusion, there is a need in the art for developing novel means and methods for biological insecticides, bioinsecticides, and biopesticides.

### SUMMARY OF THE INVENTION

According to the present invention this object is solved by providing a *protein* selected from
- a protein comprising an amino acid sequence of SEQ ID NO. 1 or an amino acid sequence having at least 60% sequence identity, preferably at least 70% sequence identity, more preferably at least 80% or 81% sequence identity to the amino acid sequence of SEQ ID NO. 1, and/or
- a protein encoded by a nucleotide sequence of SEQ ID NO. 2 or a nucleotide sequence having at least 60% sequence identity, preferably 70% sequence identity, more preferably 80% or 81% sequence identity to the nucleotide sequence of SEQ ID NO. 2.

According to the present invention this object is solved by providing a *nucleic acid molecule*, comprising a nucleotide sequence of SEQ ID NO. 2 or a nucleotide sequence having at least 60% sequence identity, preferably 70% sequence identity, more preferably 80% or 81% sequence identity to the nucleotide sequence of SEQ ID NO. 2.

According to the present invention this object is solved by providing a *host cell*, containing a nucleic acid molecule of the present invention and/or expressing the protein of the present invention.

According to the present invention this object is solved by providing a recombinant protein obtained from a host cell of the present invention.

According to the present invention this object is solved by providing a *plant* or *fungus*, containing a nucleic acid molecule according the present invention and/or expressing the protein of the present invention and/or comprising host cell(s) of the present invention.

According to the present invention this object is solved by using
- the protein of the present invention,
- the nucleic acid molecule of the present invention,
- the host cell of the present invention, and/or
- the plant or the fungus of the present invention,
as bioinsecticide.

According to the present invention this object is solved by providing a (bio)insecticide or (bio)pesticide composition comprising
(a) a protein, a nucleic acid molecule and/or a host cell of the present invention, and
(b) excipient(s) and/or carrier.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "1 to 21" should be interpreted to include not only the explicitly recited values of 1 to 21, but also include individual values and sub-ranges within the indicated range. Thus, included in this numerical range are individual values such as 1, 2, 3, 4, 5 .... 17, 18, 19, 20, 21 and sub-ranges such as from 2 to 10, 8 to 15, etc. This same principle applies to ranges reciting only one numerical value, such as "at least 80%" or "at least 82%". Furthermore, such an interpretation should apply regardless of the breadth of the range or the characteristics being described.

### Ageritin and respective nucleic acid molecules encoding it

As discussed above, the present invention provides the fungal protein ageritin.

In particular, the present invention provides a *protein* selected from
- a protein comprising an amino acid sequence of SEQ ID NO. 1 or an amino acid sequence having at least 60% sequence identity to the amino acid sequence of SEQ ID NO. 1, and/or
- a protein encoded by a nucleotide sequence of SEQ ID NO. 2 or a nucleotide sequence having at least 60% sequence identity to the nucleotide sequence of SEQ ID NO. 2.

In one embodiment, the present invention provides a protein that consists of the amino acid sequence of SEQ ID NO. 1.

In one embodiment, the present invention provides a protein that comprises an amino acid sequence having at least 70% sequence identity, more preferably at least 80% sequence identity, more preferably at least 81% sequence identity, more preferably at least 82% sequence identity, more preferably at least 83% sequence identity, more preferably at least 84% sequence identity, more preferably at least 85% sequence identity, more preferably at least 90%, more preferably at least 92%, more preferably at least 95%, more preferably at least 96% sequence identity, more preferably at least 97% sequence, more preferably at least 98% sequence identity or more preferably 99% sequence identity to the amino acid sequence of SEQ ID NO. 1

In one embodiment, the present invention provides a protein that is encoded by a nucleotide sequence that consists of the nucleotide sequence of SEQ ID NO. 2.

In one embodiment, the present invention provides a protein that is encoded by a nucleotide sequence having at least 70% sequence identity, more preferably at least 80% sequence identity, more preferably at least 81% sequence identity, more preferably at least 82% sequence identity, more preferably at least 83% sequence identity, more preferably at least 84% sequence identity, more preferably at least 85% sequence identity, more preferably at least 90%, more preferably at least 92%, more preferably at least 95%, more preferably at least 96% sequence identity, more preferably at least 97% sequence, more preferably at least 98% sequence identity or more preferably 99% sequence identity to the nucleotide sequence of SEQ ID NO. 2.
SEQ ID NO. 1 Ageritin (wt) amino acid sequence (verified protein sequence encoded by gene *AaeAGT1*, gene ID AAE3_01767): 
MSESSTFTTAVVPEGEGVAPMAETVQYYNSYSDASIASCAFVDSGKDIDKTKLVTYTSRLA
ASPAYQKVVGVGLKTAAGSIVPYVRLDMDNTGKGIHFNATKLSDSSAKLAAVLKTTVSMTEA
QRTQLYMEYIKGIENRSAQFIWDWWRTGKAPA
SEQ ID NO. 2 Ageritin (wt) nucleotide sequence (verified cDNA sequence of gene *AaeAGT1*, gene ID AAE3_01767)

atgtccgagtcctctaccttcaccactgcggtagtacctgaaggcgaaggagttgctccaat
ggcagagaccgtgcagtattacaactcctactctgacgcatccatcgcgtcttgcgcatttg
tagactcggggaaggacaaaattgataagaccaagttggtcacgtacaccagccgcctcgcc
gcaagccccgcatatcagaaggtcgtcggcgtcggcctcaaacggccgcgggctccatcgt
gccctacgtccggctcgacatggacaacaccggcaagggcatccatttcaacgcgactaaac
tctccgacagttccgccaagctcgccgcggtgctcaagacgacggtgtccatgaccgaggca
cagcgaactcaactctacatggagtatatcaagggcatcgagaatcggagtgcgcagtttat
ttgggactggtggaggacgggcaaggctccggcgtga

In one embodiment, the protein of the present invention comprises an amino acid substitution in position Y57, D91 and/or K110.

Preferably, the protein of the present invention does not comprise an amino acid substitution in position R87, D89 and/or H98.

As discussed above, the present invention provides nucleic acid molecules.

In particular, the present invention provides a *nucleic acid molecule*, comprising a nucleotide sequence of SEQ ID NO. 2 or a nucleotide sequence having at least 60% sequence identity to the nucleotide sequence of SEQ ID NO. 2,
preferably coding for a protein according to the present invention.

In one embodiment, the present invention provides a nucleic acid molecule consisting of the nucleotide sequence of SEQ ID NO. 2.

In one embodiment, the present invention provides a nucleic acid molecule that comprises a nucleotide sequence having at least 70% sequence identity, more preferably at least 80% sequence identity, more preferably at least 81% sequence identity, more preferably at least 82% sequence identity, more preferably at least 83% sequence identity, more preferably at least 84% sequence identity, more preferably at least 85% sequence identity, more preferably at least 90%, more preferably at least 92%, more preferably at least 95%, more preferably at least 96% sequence identity, more preferably at least 97% sequence, more preferably at least 98% sequence identity or more preferably 99% sequence identity to the nucleotide sequence of SEQ ID NO. 2.

In one embodiment, the nucleic acid molecule of the present invention further comprises vector nucleic acid sequences, preferably expression vector sequences.

In one embodiment, the nucleic acid molecule of the present invention further comprises promoter nucleic acid sequences and terminator nucleic acid sequences, and/or comprises other regulatory nucleic acid sequences.

In one embodiment, the nucleic acid molecule of the present invention comprises dsDNA, ssDNA, cDNA, LNA, PNA, CNA, RNA or mRNA or combinations thereof.

### Host cells, plants and fungi expressing ageritin

As discussed above, the present invention provides host cells.

In particular, the present invention provides a *host cell* which contains a nucleic acid molecule according to the present invention and preferably expresses said nucleic acid molecule, and/or expresses the protein according to the present invention.

The host cell is preferably selected from a bacterial cell, a plant cell or a fungal cell.

In one embodiment, the host cell is a bacterial cell:
preferably an *Escherichia* cell,
such as *Escherichia coli*
   e.g. *E. coli* K12, DH5alpha, JM101, JM109, BL21, SURE,

In one embodiment, the host cell is a plant cell,
preferably from an agricultural crop and/or an ornamental plant,
such as *Zea mays* (field corn: dent corn, flint corn, flour corn and blue corn; sweet corn: *Zea mays* convar. *saccharata* var. *rugosa*; popcorn: *Zea mays everta*), *Gossypium* spp. (cotton species), *Capsicum* spp., *Solanum tuberosum, Solanum lycopersicum, Nicotiana tabacum, Phaseolus lunatus, Pisum sativum* var. *macrocarpon, Glycine max, Arachis hypogaea, Triticum aestivum, Avena sativa, Hordeum vulgare, Secale cereale, Malus domestica, Pyrus communis, Prunus* spp., e.g. *P. avium, P. persica, and P. domestica, Ribes* spp., e.g. *R. uva-crispa, R. nigrum,* and *R. rubrum, Vitis vinifera.*

In one embodiment, the host cell is a fungal cell,
preferably
- from an edible, medicinal or ornamental (cultivated) mushroom,
   such as *Agaricus bisporus, Pleurotus ostreatus, P. eryngii, Lentinula edodes, Hericium* spp., *Volvariella volvacea, Grifolafrondosa, Ganoderma* spp., *Trametes* spp, *Auricularia polytricha*, *Flammulina velutipes*, *Lentinus (Pleurotus) sajor-caju, Hypsizygus tessellatus* (Buna-shimeji, Brown Beech Mushroom, and Bunapi-shimeji, White Beech Mushroom)
- a yeast cell, preferably a basidiomycete yeast cell,
   such as *Ustilago* spp., e.g. *U. maydis, Microbotryum* spp., e.g. *M. lychnidis-dioicae,* and *M. violaceum*, *Xanthophyllomyces dendrorhous*, *Rhodotorula* spp., e.g. *R. glutinis, and R. mucilaginosa, Sporobolomyces* (*Sporidiobolus*) spp., *e.g. S. roseus, Mrakia* spp., e.g. *M. frigida, M. psychrophila,* and *M. gelida,*
- from an entomopathogenic or mite-pathogenicfungus,
   such as *Beauveria* (*Cordyceps*) *bassiana, Hirsutella thompsonii, Isaria* (*Paecilomyces*) spp., e.g. *I. fumosorosea*, *Lecanicillium* spp., e.g. *L. longisporum*, *and L. muscarium*, *Metarhizium* spp., e.g. *M. anisopliae*, and *M. acridum, Nomuraea* spp., e.g. *N. rileyi.*

The fungal cell according to the present invention is not a cell from *A. aegerita*, the fungus where ageritin is derived from.

The present invention provides a recombinant protein which is obtained from a host cell of the present invention, wherein the host cell is preferably a cell from *Escherichia coli, Zea mays, Gossypium* spp. (cotton species), *Capsicum* spp., *Solanum tuberosum, Solanum lycopersicum, Nicotiana tabacum, Phaseolus lunatus, Pisum sativum* var. *macrocarpon, Glycine max, Arachis hypogaea, Triticum aestivum, Avena sativa, Hordeum vulgare, Secale cereale, Malus domestica, Pyrus communis, Prunus* spp., e.g. *P. avium, P. persica, and P. domestica, Ribes* spp., e.g. *R. uva-crispa, R. nigrum,* and *R. rubrum, Vitis vinifera,*
more preferably *Escherichia coli, Zea mays, Gossypium* spp. (cotton species), *Capsicum* spp., *Solanum tuberosum, Solanum lycopersicum, Nicotiana tabacum, Phaseolus lunatus, Pisum sativum* var. *macrocarpon, Glycine max, Arachis hypogaea, Triticum aestivum, Avena sativa, Hordeum vulgare, Secale cereale, Malus domestica, Pyrus communis, Prunus avium, Prunus persica, Prunus domestica, Ribes uva-crispa, Ribes nigrum, Ribes rubrum, Vitis vinifera.*

In one embodiment, the ageritin of the present invention is recombinantly produced in the cytoplasm of the host cell, such as *E. coli.* Such recombinant ageritin lacks posttranslational modifications (glycosylations). Furthermore, it preferably shows a higher thermal and chemical stability and a high mutational adaptability.

As discussed above, the present invention provides plants.

In particular, the present invention provides a *plant*, which
contains a nucleic acid molecule according to the present invention and preferably expresses said nucleic acid molecule,
and/or expresses the protein according to the present invention,
and/or comprises host cell(s) of the present invention,

Preferably, the plant is an agricultural crop and/or an ornamental plant,
such as *Zea mays* (field corn: dent corn, flint corn, flour corn and blue corn; sweet corn: *Zea mays* convar. *saccharata* var. *rugosa*; popcorn: *Zea mays everta*), *Gossypium* spp. (cotton species), *Capsicum* spp., *Solanum tuberosum, Solanum lycopersicum, Nicotiana tabacum, Phaseolus lunatus, Pisum sativum* var. *macrocarpon, Glycine max, Arachis hypogaea, Triticum aestivum, Avena sativa, Hordeum vulgare, Secale cereale, Malus domestica, Pyrus communis, Prunus* spp., e.g. *P. avium, P. persica, and P. domestica, Ribes* spp., e.g. *R. uva-crispa, R. nigrum,* and *R. rubrum, Vitis vinifera.*

Preferably, the plant of the present invention is protected against insect pests.

For example, a *Zea mays* plant of the present invention is protected against the European corn borer (*Ostrinia nubilalis*).

In one embodiment, the plant furthermore comprises *Bacillus thuringiensis*, or *Bacillus thuringiensis* subspecies *israelensis* (*Bti*).

As discussed above, the present invention provides fungi.

In particular, the present invention provides a *fungus*, which
contains a nucleic acid molecule according to the present invention and preferably expresses said nucleic acid molecule,
and/or expresses the protein according to the present invention,
and/or comprises host cell(s) of the present invention,

Preferably, the fungus is an edible, medicinal or ornamental (cultivated) mushroom, such as *Agaricus bisporus*, *Pleurotus ostreatus*, *P. eryngii*, *Lentinula edodes*, *Hericium* spp., *Volvariella volvacea*, *Grifola frondosa*, *Ganoderma* spp., *Trametes* spp., *Auricularia polytricha*, *Flammulina velutipes*, *Lentinus (Pleurotus) sajor-caju*, *Hypsizygus tessellatus* (Buna-shimeji, Brown Beech Mushroom, and Bunapi-shimeji, White Beech Mushroom).

Preferably, the fungus of the present invention is protected against insect pests, such as fungus gnats and fungus pests.

For example, fungus gnats (Sciaridae and Phoridae), such as *Lycoriella ingenua*, are black flies which damage the fruiting bodies grown in mushroom farms, e.g. of *Agaricus bisporus*.

Further examples of fungus pests are:
- Sciaridae/Phoridae feeding on Volvariella volvacea (Indian J Microbiol (Apr-June 2011) 51(2):200-205)
- "Flies" feeding on Pleurotus ostreatus (Rev Argent Microbiol. 2018 Apr - Jun;50(2):216-226)
- Camptomyia corticalis and C. heterobia feeding on Lentinula edodes (Mol Ecol Resour. 2013 Mar; 13(2):200-9.)
- Brennandania lambi feeding on Agaricus bisporus (Exp Appl Acarol. 2001;25(3):187-202.)
- Tyrophagus putrescentiae (storage mite) feeding on Agaricus bisporus, Pleurotus ostreatus, Auricularia polytricha and Flammulina velutipes (Environ Entomol. 2015 Apr;44(2):392-9.)

In one preferred embodiment, the fungus is an entomopathogenic or mite-pathogenic fungus, such as such as *Beauveria* (*Cordyceps*) *bassiana*, *Hirsutella thompsonii*, *Isaria* (*Paecilomyces*) spp., e.g. *I. fumosorosea*, *Lecanicillium* spp., e.g. *L. longisporum*, *and L. muscarium*, *Metarhizium* spp., e.g. *M. anisopliae*, and *M. acridum*, *Nomuraea* spp., e.g. *N. rileyi.*
e.g. *Beauveria bassiana, Isaria fumosorosea. Nomuraea rileyi, Hirsutella thompsonii, Metarhizium anisopliae, Lecanicillium longisporum.*

The fungus according to the present invention is not *A. aegerita,* i.e. the fungus where ageritin is derived from.

### Uses as (bio)insecticides

As discussed above, the present invention provides the use of
- the protein of the present invention,
- the nucleic acid molecule of the present invention,
- the host cell of the present invention, and/or
- the plant or the fungus of the present invention,
as bioinsecticide.

The term "bioinsecticide" as used herein refers to biological insecticides, which are generated by organisms, such as plants or fungi, which express insecticidal compounds or components, such as insecticidal proteins, metabolites.

In a preferred embodiment, the use is directed against mosquitoes, preferably disease vector mosquitoes, such as of the viral diseases yellow fever, chikungunya, dengue, Zika, west nile fever, parasitic diseases such as lymphatic filariasis and malaria),
more preferably
*Aedes* spp. (*Aedes aegypti, Ae. japonicus, Ae. albopictus*),
*Culex* spp. and *Culexpipiens* spp. (*C*. *nigripalpus, C. pipiens, C. tarsalis, C. annulirostris, C. univittatus, C. quinquefasciatus),*
*Anopheles* spp. (*Anopheles quadrimaculatus, Anopheles gambiae, Anopheles stephensi, Anopheles maculipennis, Anopheles funestus, Anopheles arabiensis)*,
*Stegomyia (Aedes) aegypti,*
*Stegomyia (Aedes) albopicta,*
*Aedimorphus (Aedes) vexans.*

More preferably, the use is directed against the larvae of said mosquitoes ("larviciding").

In said embodiment, the use also contributes to the increase of live quality in the environment of water bodies or inshore waters, since said water bodies or inshore waters will no longer be the breeding ground of mosquitoes.

Toxicity assays revealed a strong larvicidal activity of ageritin, as shown herein, the cytoplasmically synthesized ribotoxin from the mushroom *Agrocybe aegerita*, against mosquito larvae making this protein an excellent novel biopesticide/bioinsecticide.

Tropical mosquito species like the yellow fever mosquito *Aedes aegypti* are most important disease vectors for the most important infectious diseases globally, including the severe arboviral diseases such as yellow fever, chikungunya, dengue or zika. Stopping the development from larvae into adult mosquitos by larviciding (killing of their larvae) efficiently can abolish the establishment and spreading of stable populations by such mosquitos and can hence minimize the risk of disease transmission.

Larviciding is currently done by introducing the biopesticide/bioinsecticide *Bacillus thuringiensis israelensis* (*Bti*) into bodies of standing water reservoirs as potential sites of egg laying and larval hatching. However, this practice is potentially at risk of becoming ineffective as different genetic reports on *Bti* resistance development by *Ae. aegypti* have been published (see e.g. Cadavid-Restrepo *et al.,* 2012). Ageritin is therefore a vital alternative to *Bti* not least as a reserve larvicidal in cases where *Bti* becomes ineffective. In addition, *Bti* containing products on the market are known for getting less available to the larvae over time, e.g. due to sinking down the water column or due to being uptaken by other organisms for which they are not toxic.

A prerequisite for the use of Ageritin as bioinsecticide is, besides its potency against target insects, its lack of side effects i.e. toxicity towards other cells or organisms. Thus far, we can say that the protein is toxic for insects such as *Ae. aegypti,* CNS model cell lines but not for nematodes including *Caenorhabditis elegans.* In addition, the mushroom *A. aegerita* where Ageritin is derived from, is a commercially cultivated edible mushroom.

Larviciding for mosquito control is conventionally done on a global scale by *Bacillus thuringensis israelensis (Bti)* treatment, not least in Switzerland. However, this conventional practice is potentially at risk of becoming ineffective as cases of resistance development to *Bti* are reported in pestiferous mosquito species such as *Ae. aegypti* (see e.g. Cadavid-Restrepo *et al.,* 2012). In cases where *Bti* becomes ineffective due to a potential establishment of *Bti* resistant *Ae. aegypti* populations, Ageritin is a valuable and highly effective alternative.

In addition, other important disease vector mosquitos on the rise like the common malaria mosquito *Anopheles quadrimaculatus* in the United States of America, are naturally more difficult to control with *Bti.* For example, *Bti* applied as a liquid is heavy and sinks into the water column which makes it less available to surface feeding larvae like the ones of *An*. *quadrimaculatus* (Weeks *et al.,* 2018). Ageritin is a small highly soluble protein which should not be so prone to sink into the water column and should thus be more available also to surface feeding larvae like to ones of *An. quadrimaculatus.*

Moreover, *Bti* toxicity against for example *Ae. aegypti* is mediated by synergism effects between the different kinds of crystal proteins (*Bti* toxins) which are produced during sporulation in *Bti.* The insecticidal activity of the isolated *Bti* toxins is magnitudes of order less toxic than the crystal inclusion containing all toxins (Canton *et al.,* 2011). In contrast to that, Ageritin is highly toxic to *Ae. aegypti* on its own.

Applications of other fungal ribotoxins as biopesticides have been suggested (Olombrada *et al*.,Toxins 2017). In contrast to these secreted ribotoxins, however, Ageritin is the first and so far only intracellular ribotoxin produced in the cytoplasm. This feature makes it much easier to produce this toxin recombinantly such as in *E. coli* and to keep the toxin associated with the bacterial cells if desired for the specific application.

In one embodiment, the use is in combination with further insecticide(s) against mosquito larvae, such as
methionine
temephos
*Bacillus thuringiensis* subspecies *israelensis* toxins (*Bti* toxins),
*Lysinibacillus sphaericus* powder SPH88,
chitin synthesis inhibitor(s), e.g. diflubenzuron, novaluron,
pyriproxyfen,
methoprene,
anethole,
cinnamaldehyde,
cinnamyl acetate,
or combinations thereof.

Examples of (bio)pesticides against mosquito larvae:
*Bacillus thuringiensis* subspecies *israelensis* toxins (*Bti* toxins) are a mix of crystal (Cry) proteins expressed during *B. thuringiensis* subsp. *israelensis* sporulation. Spores mainly contain: Cry11Aa, Cry4Aa, Cry4Ba and CytlAa (Berry *et al.,* 2002).
*Lysinibacillus sphaericus* powder SPH88 (Pasteur Institute, Paris, France, serotype H5a5b strain 2362; see Suter *et al.,* 2017).

Vectomax CG® (Valent Biosciences Corporation, Libertyville, IL, USA) is a commercial product available as water-soluble pouches containing a granular formulation that combines 4.5% Bti (serotype H-14, strain AM65-52) and 2.7% *L. sphaericus* (2362, serotype H5a5b, strain ABTS 1743) spores and insecticidal crystals as active ingredients (see Suter *et al.,* 2017).

Methionine, see Weeks *et al.* (2018).
Temephos, see Suter *et al.* (2017).
Pyriproxyfen, see Suter *et al.* (2017).

Diflubenzuron and novaluron, both are chitin synthesis inhibitors. Diflubenzuron can be used together with *Bti*, see Suter *et al.* (2017).

Anethole shows larvicidal activity against *Aedes aegypti,* see Cheng *et al.* (2004). Cinnamaldehyde shows larvicidal activity against *Aedes aegypti* larvae, see Cheng *et al.* (2004). Cinnamyl acetate shows larvicidal activity against *Aedes albopictus* larvae, see Cheng *et al.* (2009).

In a preferred embodiment, the use is directed against insect pests attacking crop plants and/or ornamental plants,
such as
- *Lepidoptera,*
   such as *Ostrinia nubilalis* (European corn borer), *Spodoptera exigua*, *S. frugiperda, S. littoralis, Pectinophora gossypiella, Heliothis virescens, Helicovera zea, Chrysodeixis includes,Helicoverpa armigera,* cutworms (various species including *Agrotis* spp., *Peridroma saucia, Nephelodes minians*), hornworms (*Manduca quinquemaculata*, *Manduca sexta)*, Lepidoptera complex (e.g. rice stem borers *Chilo auricilius*, *C. suppressalis*, *Scirpophaga incertulas, S. innotata*), *Grapholita funebrana, Phthorimaea operculella, Elasmopalpus lignosellus*),
- *Coleoptera,*
   such as Colorado potato beetle (*Leptinotarsa decemlineata*), *Diabrotica spp., Anthonomus grandis* (boll weevil), *Oulema melanopus*, *O. gallaeciana*
- sucking insects
   such as Thrips (Thysanoptera, e.g. *Thrips tabaci, Frankliniella schultzei),* Aphids (Hemiptera, e.g. *Aphis gossypii, Dysdercus koenigii, Creontiades dilutes*), white flies (Homoptera),
- Arachnida
   such as *Tetranychus urticae, Panonychus ulmi* (European red mite).

In one embodiment, said use is in combination with further insecticide(s), such as
*Bacillus thuringiensis* toxins (*Bt* toxins),
azadirachtin,
chitin synthesis inhibitor(s), e.g. diflubenzuron,
tebufenozide,
methoprene,
malathion,
or combinations thereof.

Examples of (bio)pesticides against insect pests attacking crop plants:
*Bacillus thuringiensis* toxins (*Bt* toxins) are mediated (mainly) by different crystal proteins expressed by *B. thuringiensis.* Approved *Bt* genes include single and stacked (event names bracketed) configurations of:
Cry1A.105 (MON89034)
CryIAb (MON810)
CryIF (1507)
Cry2Ab (MON89034)
Cry3Bb1 (MON863 and MON88017)
Cry34Ab1 (59122)
Cry35Ab1 (59122)
mCry3A (MIR604)
Vip3A (MIR162).

Azadirachtin is used against *Bt* toxin resistant pests, such as the African cotton leafworm *Spodoptera littarolis.*

Diflubenzuron is used for killing fungus gnat larvae in greenhouse soil.

Tebufenozide is used to control caterpillar pests.

In one embodiment, the use is
- against mite (Acaridae),
   such as against *Brennandania lambi, Tyrophagus putrescentiae* (storage mite), *Panonychus ulmi* (European red mite), *Tetranychus urticae,*
- against fungus gnats and fungus pests,
   such as against Sciaridae and Phoridae (such as *Lycoriella ingenua), Camptomyia corticalis, C. heterobia,* "flies",
- against storage pests,
   such as Bruchids [*Callosobruchus chinensis* (L.) and *Callosobruchus maculatus* (F.)], *Caryedon serratus*, storage insect pests, including the Indian meal moth, *Plodia interpunctella* (Hubner) (Lepidoptera: Pyralidae); *Sitophilus spp.* (Coleoptera: Curculionidae); and their natural enemies [e.g., *Cephalonomia tarsalis* (Ashmead) (Hymenoptera: Bethylidae), and *Anisopteromalus calandrae* (Howard) (Hymenoptera: Pteromalidae)],
- -in hygiene,
   such as against cockroaches, ants, termites and bed bugs,
   and/or
- in crop protection,
   such as against slugs, blossom beetle, rape stem beetle, Aphids, *Drosophila susukii,* raspberry weevil, caterpillars, fungus gnats, European grape vine moth, stem borers, European red mite (*Panonychus ulmi*),
wherein the use can be in combination with further insecticide(s), such as insecticides disclosed herein.

For example, the use against mites can be in combination with Permethrin.

### Bioinsecticide compositions

As discussed above, the present invention provides a (bio)insecticide composition.

Said composition comprises
(a) a protein of the present invention, a nucleic acid molecule of the present invention and/or a host cell of the present invention, and
(b) excipient(s) and/or carrier.

In one embodiment, the composition furthermore comprises:
(c) further active agent(s),
such as insecticide(s) or pesticide(s).

Examples for further active agent(s) are (bio)pesticides against mosquito larvae and/or (bio)pesticides against insect pests attacking crop plants, as described above, such as:
methionine
temephos
*Bacillus thuringiensis* subspecies *israelensis* toxins (*Bti* toxins),
*Lysinibacillus sphaericus* powder SPH88,
chitin synthesis inhibitors, e.g. diflubenzuron, novaluron,
pyriproxyfen,
methoprene,
anethole,
cinnamaldehyde,
cinnamyl acetate,
*Bacillus thuringiensis* toxins (*Bt* toxins),
azadirachtin,
tebufenozide,
malathion,
or combinations thereof.

In one embodiment, the composition is formulated as
a solution, such as liquids e.g. liquid concentrates,
a powder (such as a wettable powder),
granules,
baits.

Liquid concentrates and powders can be used as spray.

The formulations can further comprise spreading and/or sticking agent(s).

### Preferred embodiments

In the present study, we identified the ageritin-encoding gene *AaeAGT1* and the neighboring gene *AaeAGT2* on the chromosomal *A. aegerita* DNA encoding a paralogous protein, from the recently published genome sequence of *A. aegerita* (Gupta *et al.,* 2018). Transcriptional profiling of *AaeAGT1* revealed a very strong induction of *AaeAGT1* expression during mushroom formation with a trend towards specificity to the developing mushroom cap. The predicted amino acid sequence of ageritin revealed marked differences to all other fungal ribotoxins described so far. Employing heterologous expression of ageritin in *E. coli,* we subsequently checked for insect and nematode toxicity as well as for *in vitro* ribonucleolytic activity of wild type and mutagenized ageritin. Finally, we functionally characterized the *AaeAGT2-encoded* ageritin paralog, which we identified based on its high sequence similarity to AaeAgt1. Searching for homologies of sequences we found that homologs of ageritin are widespread among a variety of fungal species including several plant pathogenic fungi.

### - Abstract

Here, we report on a ribotoxin from *Agrocybe aegerita* referred to as ageritin, the first ribotoxin described from Basidiomycota. The amino acid sequence markedly differs from Ascomycota ribotoxins. It does not contain any signal peptide for classical secretion, which represents the first cytoplasmic ribotoxin. Ageritin-encoding gene *AaeAGT1* is highly induced during fruiting. *AaeAGT1-cDNA* was cloned and expressed in *E. coli* for further study. Toxicity assays showed a strong insecticidal activity against *Aedes aegypti* whereas no toxicity was found against nematodes. The rRNase activity of the ageritin was confirmed *in vitro* using lysate ribosomes. Mutagenesis studies showed correlation between *in vivo* and *in vitro* activities indicating that the entomotoxicity is mediated by the ribonucleolytic cleavage. The strong toxicity of ageritin against mosquito larvae makes it a new biopesticide.

### - Results

### 1. Identification of the ageritin encoding gene from the A. aegerita genome sequence

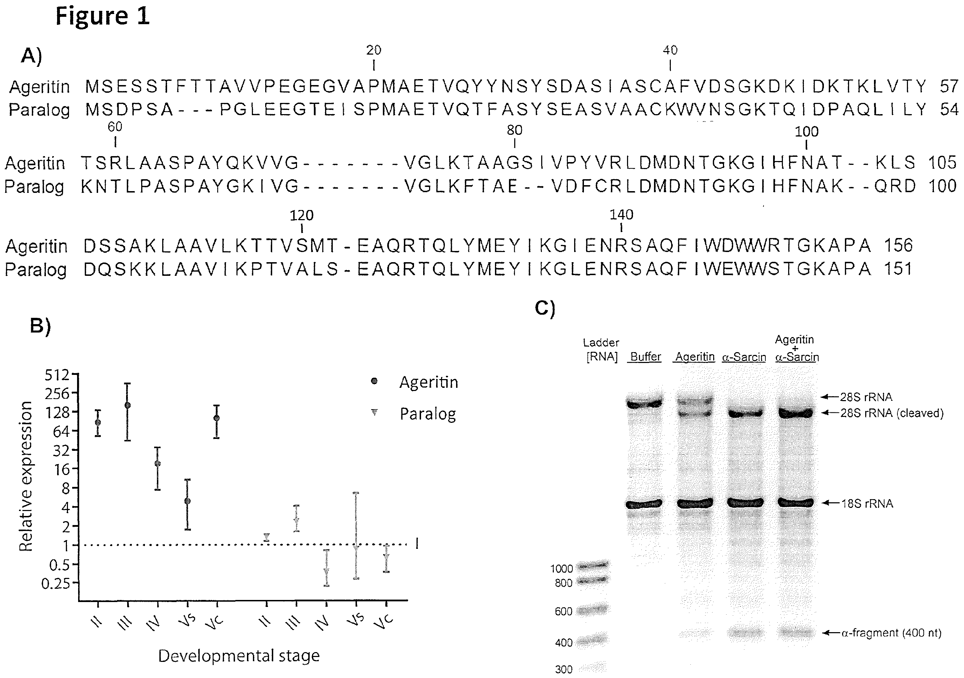

By BLASTing the published 25 N-terminal residues of ageritin (Landi *et al.,* 2017) against the predicted proteome of *A. aegerita*, we were able to identify the ageritin-encoding gene from the fungus. It will be referred to as *AaeAGT1* (gene ID AAE3_01767). In addition, we found a neighboring gene on the chromosomal DNA of *A. aegerita* encoding a paralogous protein, which is referred as *AaeAGT2* (gene ID AAE3_01768). Compared to ageritin, the deduced amino acid sequence of AaeAgt2 displays 63% sequence identity (Figure 1A). Interestingly, the predicted amino acid sequence of AaeAgt1 neither shows sequence similarity to any of the known ascomycete ribotoxins nor does it possess a known signal sequence for classical secretion, suggesting a novel type of ribotoxin.
SEQ ID NO. 1 shows the Ageritin (wt) amino acid sequence, the verified protein sequence encoded by gene *AaeAGT1,* gene ID AAE3_01767.
SEQ ID NO. 2 shows the Ageritin (wt) nucleotide sequence, the verified cDNA sequence of gene *AaeAGT1,* gene ID AAE3_01767.
SEQ ID NO. 3 shows the amino acid sequence of AaeAgt2: 
MSDPSAPGLEEGTEISPMAETVQTFASYSEASVAACKWVNSGKTQIDPAQLILYKNTLPASP
AYGKIVGVGLKFTAEVDFCRLDMDNTGKGIHFNAKQRDDQSKKLAAVIKPTVALSEAQRTQL
YMEYIKGLENRSAQFIWEWWSTGKAPA
SEQ ID NO. 4 shows the nucleotide sequence of *AaeAGT2*

atgtctgacccatccgcacccggactcgaagagggcaccgaaatctcacccatggccgagac
ggtgcagaccttcgcgagctactccgaggcctccgttgccgcttgcaagtgggtcaactcgg
gaaagacccagattgacccagcccagctcatcctgtacaagaacaccctccccgcaagcccc
gcatacggcaaaatcgtcggcgtcggcctaaaattcaccgccgaggtcgacttctgccgact
cgacatggacaacacaggcaagggcatccacttcaacgccaagcagcgggacgaccagtcca
agaagctcgcggcggtgatcaagccgactgttgcgttgagcgaggcccagcgcacgcagctg
tacatggagtatatcaaggggctcgagaacaggagtgcgcagtttatttgggagtggtggag
caccggaaggcaccggcgtga

### 2. Expression of reference genes during A. aegerita vegetative growth and mushroom formation

Three genes with a putative high expression stability during vegetative mycelium growth as well as throughout the fruiting body development have been identified within the genome sequence of this dikaryotic strain (Gupta *et al.,* 2018):
*AaeIMP1* (gene ID AAE3_02268),
*AaeTIF1* (gene ID AAE3_07769) and
*AaeARP1* (gene ID AAE3-11594).

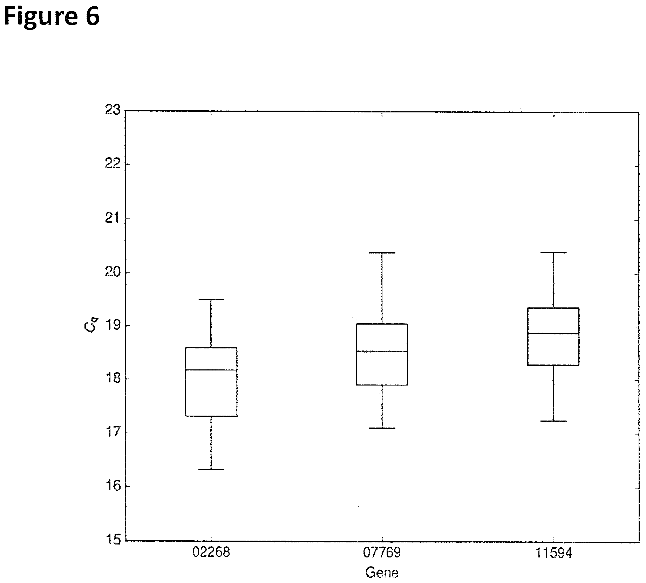

Blast search for the deduced amino acid sequences against the UniProt database (www.uniprot.org) revealed AAE3_02268 encoding a putative importin, AAE3_07669 coding for a putative translation initiation factor and AAE3_11594 encoding a putative autophagy related protein. The manually designed primer pairs for each putative reference gene showed efficiencies of 104%, 109% and 107% with a regression coefficient of 0.998, 0.996 and 0.998 for *AaelMP1*, *AaeTIF1* and *AaeARP1*, respectively. Validation of the reference genes according to their Cq-values for 12 analyzed samples (Figure 6) with the NormFinder algorithm revealed values of 0.119, 0.126 and 0.167 for genes *AaeTIF1*, *AaeIMP1* and *AaeARP1*, respectively. geNorm showed a similar trend with values of 0.29 for *AaeTIF1* and *AaeIMP1* as well as 0.33 for *AaeARP1.* Overall, on the basis of the NormFinder and geNorm validation, the reference genes *AaeTIF1* (gene ID AAE3_07769) and *AaeIMP1* (gene ID AAE3_02268) are the best combination for qRT-PCR based transcription analyses of *A*. *aegerita* genes expressed during vegetative mycelium growth or fruiting body development.

### 3. Expression of the ageritin gene during fruiting body formation of A. aegerita

In order to assess the expression of both the ageritin encoding gene *AaeAGT1* and the paralogous gene *AaeAGT2,* quantitative real-time reverse transcription PCR (qRT-PCR) analyses were carried out using RNA from different dikaryotic developmental stages of *A*. *aegerita* AAE-3. Using vegetative mycelium as a reference, a strong upregulation of *AaeAGT1* expression was found during the whole fruiting body development of *A. aegerita* AAE-3. Upregulation was not found in the case of *AaeAGT2* (Figure 1B).

The results are shown in Fig. 1B as a relative expression compared to the expression by the vegetative mycelium (I). *AaeAGT1* expression becomes highly upregulated by a factor of 87 during the shift into the ready-to-fruit mycelium (stage II). The expression level peaks with a factor of 160 when the first macroscopically visible complex multicellular plectenchymatic structures of fruiting body development can be spotted, which are referred to as fruiting body (FB) initials (stage III). Although a less pronounced expression increase was both recorded in subsequently emerging FB primordia (stage IV, *AaeAGT1* expression upregulated by a factor of 19) and in the stipe plectenchyme of the thereafter developing young FBs (stage Vs, *AaeAGT1* expression upregulated by a factor of 5), an increased expression was again observed within the cap plectenchyme of young FBs (stage Vc, 100-fold).

*AaeAGT2* showed no clear change in expression of the magnitudes of the *AaeAGT1* amplicon varying from 0.4 in stage IV to 2.3 in stage 111. Both gene's Cq values in the calibrator stage were very similar with an average 25.55 for the *AaeAGT1* amplicon and an average 25.73 for its paralog *AaeAGT2* indicating comparable template amounts for a '1-fold' expression for the two genes in this cDNA pool.

### 4. Heterologous expression and purification of ageritin from E. coli cells

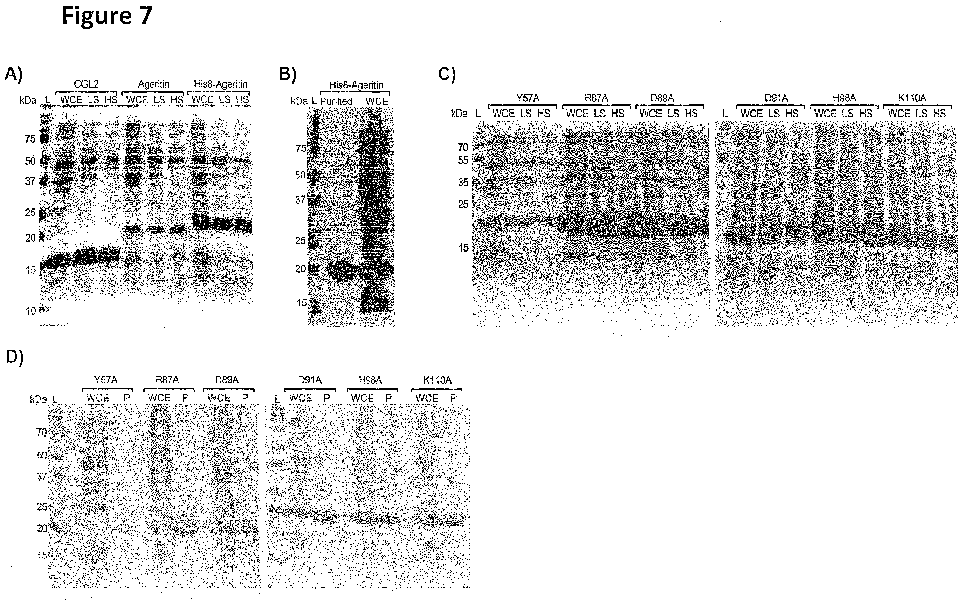

In contrast to all other ribotoxins, AaeAgt1 does not contain a signal peptide for a classical secretion and is thus predicted to be localized in the cytoplasm. Based on the lack of a signal peptide in the predicted amino acid sequence of ageritin, we expressed the protein in the cytoplasm of *E. coli.* It was shown that both, untagged and N-terminally His8-tagged ageritin were expressed in a fully soluble form in the bacterial cytoplasm (Figure 7A). The solubility of the His8-tagged ageritin allowed us to purify the recombinant protein for the *in vitro* experiments (Figure 7A). His8-ageritin was purified over Ni-NTA affinity columns to homogeneity (Figure 7B). The purified ageritin protein was used for *in vitro* ribonucleolytic cleavage assay and for toxicity assays with the insect sell line Sf21.

### 5. rRNA cleavage activity of recombinant ageritin

Ribonucleolytic activity of ageritin was assayed against ribosomes of a rabbit reticulocyte lysate. The results indicate that ageritin, same as α-Sarcin (Chan et al., 1983), acts on the 28S rRNA subunit of ribosomes and releases as classical ribotoxin cleavage product the α-fragment (Figure 1B). We observed a similar pattern on a gel for the two samples that were either treated with only α-Sarcin or simultaneously with both, α-Sarcin and ageritin, suggesting that the cleavage site for ageritin is the very close by or identical to that of α-Sarcin (Figure 1B).

### 6. Entomo- and nematotoxicity of wild type and recombinant ageritin

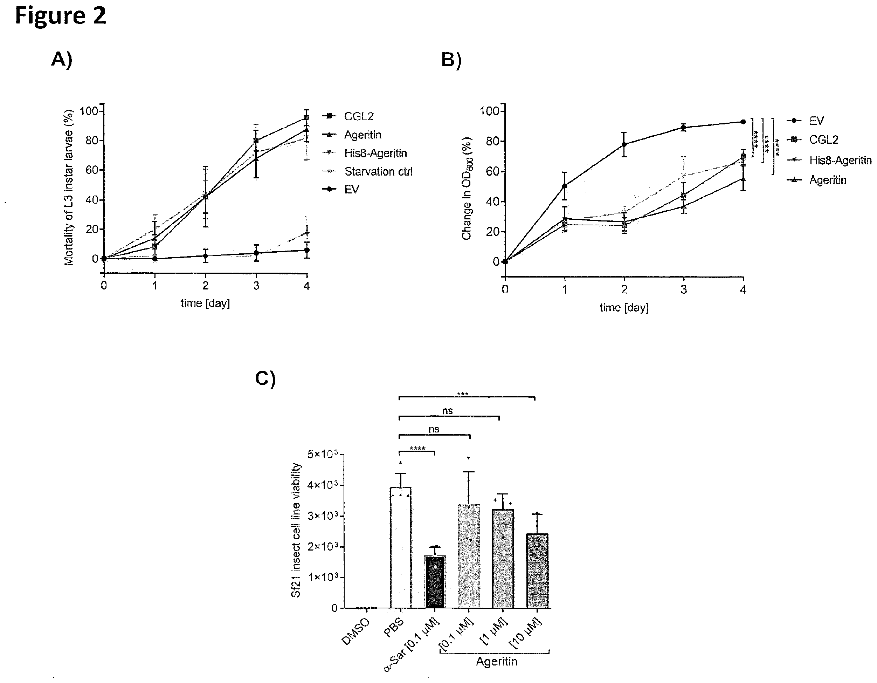

The ageritin-expressing *E. coli* cells and the tagged version were tested for insecticidal activity against L3-larvae of *A. aegypti.* After four days, significant larval mortality was recorded (Figure 2A). Concomitantly, feeding inhibition was measured with the change in OD₆₀₀ of a respective bacterial suspension. Larvae feeding on "empty vector" cells reduced the bacterial concentration resulting in a drop of OD₆₀₀. In fact, the bacterial consumption was significantly reduced in the case of Cgl2-, ageritin- or His8-ageritin-expressing bacteria (Figure 2B).

The purified ageritin protein also showed toxicity against the insect cells of Sf21 in a concentration dependent manner (Figure 2C).

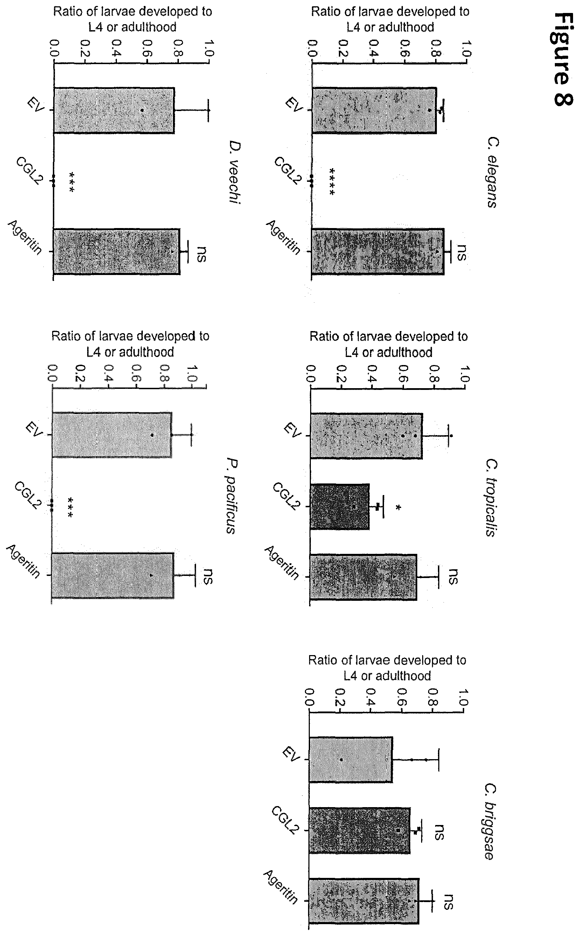

After confirming the insecticidal activity of the ribotoxin, we tested its activity against five different bacterivorous nematode species (Table 4). Synchronized L1 larvae of the nematodes (*Caenorhadbitis elegans*, *C. briggsae*, *C. tropicalis*, *Distolabrellus veechi* and *Pristionchus pacificus*) were fed with bacteria expressing ageritin, and their development was assessed after 2 days. Interestingly, ageritin was not active against any of the tested nematode species, pointing to specificity for certain organisms (Figure 8).

### 7. Correlation between rRNA cleavage and toxicity

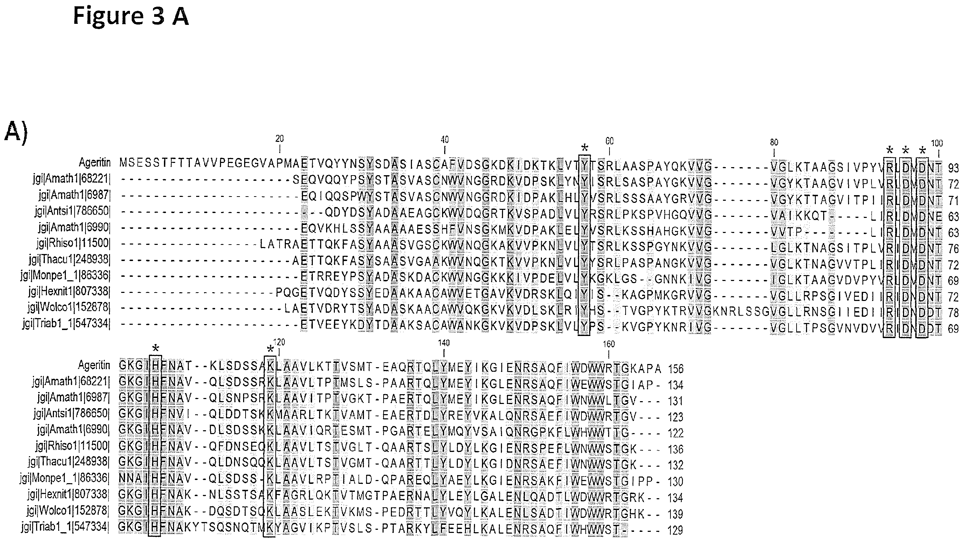

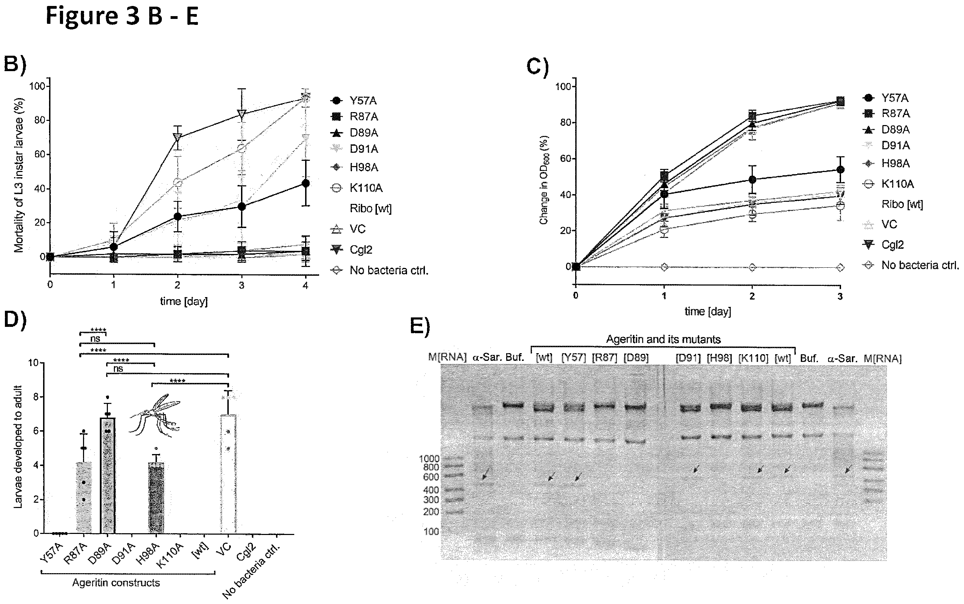

The gene sequence of ageritin was aligned against its top ten homologs. Six conserved residues were chosen for mutation into alanine (Figure 3A). All the mutants were expressible in a highly soluble manner in the cytoplasm of *E. coli* (Figure 7C). To assess the importance of the mutated residues for the insecticidal activity of ageritin, we performed a toxicity assay against *Ae. aegypti* larvae. No toxicity was measured for three conserved-site mutants (R87A, D89A and H98A) while the other three mutants (Y57A, D91A and K110A) performed similar to the wild type ageritin (Fig. 3B).

In a second approach we measured the change in OD₆₀₀ on a daily basis to check on larval feeding inhibition. The toxic constructs inhibited feeding whereas the reduction in OD₆₀₀ by non-insecticidal bacterial strains was in line with the control (Figure 3C). In order to differentiate between the effect level of the three point mutations (R87A, D89A and H98A) which had shown significant reduction in ageritin toxicity, the mosquito larvae were allowed to develop into adults. The mutant construct D89A showed complete loss of toxicity. All larvae developed into imagines (Figure 3D). An average of 40% of the larvae developed into adults when the other two conserved site mutants (R87A and H98A) were assayed. This indicates that partial toxicity in these two mutants is still present (Figure 3D).

Ageritin single-site mutant constructs were produced in *E.coli* BL21 in a fully soluble manner and purified over Ni-NTA columns (Figure 7D). Subsequently, ribonucleolytic activity of the purified proteins were assessed against ribosomes of rabbit reticulocyte lysate. Interestingly, all of the three single-site mutants which were non-toxic *in vivo* against *Ae. aegypti* larvae were also inactive in the ribosome cleavage assay (Figure 3E). The single site mutants that were still toxic *in vivo* performed similar to the wild type ageritin. This means that they all released the 400 nucleotide long ribotoxin cleavage product, the α-fragment (Figure 3E). Combined with our insecticidal assay data, the *in vitro* results demonstrate that the insecticidal toxicity is based on ribonucleolytic activity of the ageritin.

### 8. Functional comparison between ageritin and the ageritin paralog

Given the different expression dynamics of *AaeAGT2* and *AaeAGT1* throughout the developmental stages of the fungus and the recorded bioactivity spectrum of AaeAgt1, we focused on a potential bioactivity of AaeAgt2.

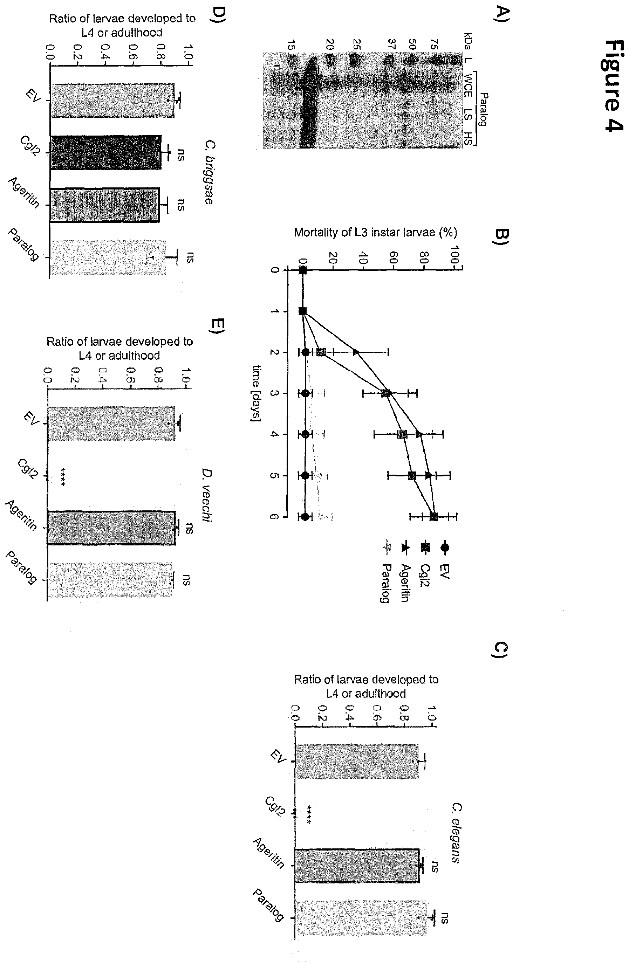

*AaeAGT2* was expressed in *E. coli* BL21 in the same manner as *AaeAGT1,* and tested against *Ae. aegypti* and bacterivorous nematodes. The ageritin paralog was also expressed as highly soluble protein in the bacterial cytoplasm (Figure 4A).

Interestingly, unlike ageritin, its paralog was not active against *Ae. aegypti* larvae (Figure 4B). Subsequently, we tested its toxicity against three different nematode species. Similar to ageritin, its paralog did not show any sign of toxicity neither against *C*. *elegans* (Figure 4C), *C. briggsae* (Figure 4D) nor *D. veechi* (Figure 4E).

This suggests a different biological function of the ageritin paralog or even non-functionality.

### 9. Distribution of ageritin homologs in the fungal kingdom

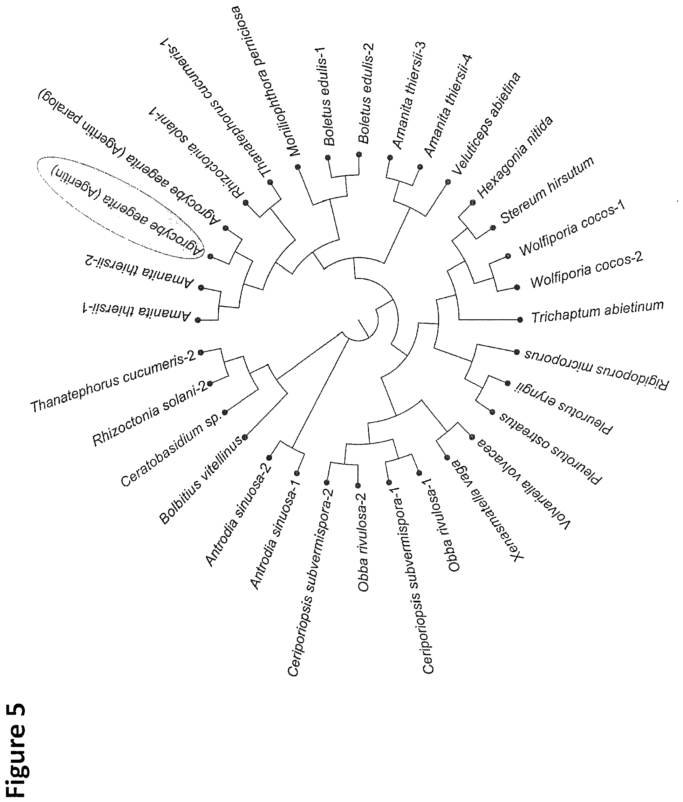

Ageritin homologs were searched using JGI MycoCosm portal (Grigoriev *et al.,* 2014). A high degree of sequence conservation was found among ageritin homologues proteins within different fungal species. The top 30 hits were retrieved from the MycoCosm portal and their phylogenetic relationships were designed as a circular cladogram (Figure 5). All 30 hits, all originate from basidiomycetes including several plant-pathogenic species such as *Rhizoctonia solani*, *Moniliophthora perniciosa, Rigidoporus microporus and Pleurotus eryngii.*

Interestingly, despite ageritin being a cytoplasmic protein, several of its homologs possess known signal sequences, which however resembles more closely the secreted ribotoxins of ascomycetes.

### - Discussion

Ribotoxins are suggested to be part of the fungal defense system against insect predators (see e.g. Olombrada *et al.,* 2013).

This is corroborated by the results of the present work, in which we report the full amino acid sequence and the gene encoding a novel type of ribotoxin named ageritin from the edible mushroom *A. aegerita.* Ageritin is so far the first ribotoxin identified from the phylum Basidiomycota. Its sequence is very different from those of the well-known ribotoxins of Ascomycota. Furthermore, it is the first cytoplasmic ribotoxin discovered up to now

Our results demonstrate high toxicity of ageritin against *Ae. aegypti* larvae, suggesting that it plays an important role as a natural defense mechanism of *A. aegerita* against insects pests of mushrooms such as fungus gnats. In cultivation facilities of the cultivated edible mushroom *Agaricus bisporus* (white button mushroom), pestiferous fungus gnats like *Lycoriella ingenua* (Sciaridae) can cause severe damages, e.g. by larval feeding on the button mushroom cultures (Cloonan *et al.,* 2016). Insecticidal activity is a common feature of fungal ribotoxins (Herrero-Galan *et al.,* 2013). Anisopilin and hirsutellin A, two previously described ribotoxins (Yang *et al.,* 1996; Herrero-Galan *et al.,* 2008) had been shown to exert cytotoxicity against insect cell lines. Similarly, ageritin showed cytotoxicity against the insect cell line Sf21, albeit at a higher concentration than the well-studied α-Sarcin, suggesting that ageritin is either less active than α-Sarcin or the reaction conditions were not optimal. The *in vitro* ribonucleolytic assay with ribosomes of rabbit reticulocyte lysate indicates that the latter is likely to be true, since, the cleavage of 28S rRNA was slower for ageritin than α-Sarcin.

Furthermore, we investigated whether the insecticidal activity of ageritin is dependent on its ribonucleolytic activity. The putative catalytic residues of ageritin could not be identified by comparison with the previously characterized ribotoxins due to the lack of sequence homology. However, previous studies had shown that catalytic residues of ribotoxins mostly consist of acidic and basic residues. The charged amino acids of ribotoxins had been found to support binding to the negatively charged rRNA facilitating interactions with target ribosomes (Herrero-Galan *et al.,* 2012). For instance, the active site of α-Sarcin consists of histidine and a glutamic acid (H50, E96 and H137) (Lacadena *et al.,* 1999). Therefore, we mutated the conserved charged amino acid residues (R87, D89, D91, H98 and K110) and tyrosine (Y57) in the ageritin amino acid sequence. Three (R87A, D89A and H98A) of the six mutations abolished both insecticidal activity and *in vitro* ribonucleolytic cleavage while the other three mutants (Y57A, D91A and K110A) did not affect neither *in vivo* nor *in vitro* activity of ageritin. The mutant studies indicate that the insecticidal activity of ageritin depends on the rRNA cleavage activity, and the residues R87, D89 and H98 are part of the catalytic site of ageritin.

While nematodes are important fungal predators in the environment, no activity of ribotoxins against nematodes has been reported so far. As ageritin differs from other ribotoxins in amino acid sequence and structure, we tested the susceptibility of five nematode species (*C*. *elegans, C. briggsae, C. tropicalis, D. veechi* and *P. pacificus*) against ageritin. However, nematotoxicity results show that ageritin is inactive, at least against these five nematode species, suggesting the ribosomes are either resistant or not accessible for ageritin. These findings are in line with previous studies on the activity spectrum of ribotoxins where they exhibit specificity to insect cells due to the special structure and composition of insect cell membrane (Olombrada et al., 2014).

Unlike ageritin, its paralog AaeAgt2 was not toxic to *Ae. aegypti* despite a 63% sequence identity with ageritin. Ageritin and its paralog differ in terms of their isoelectric point (pI) and their expression patterns during the life cycle of the mushrooms. Furthermore, it has been shown, that the positive charge of ribotoxins is crucial for the interaction with cell membranes and ribosomes (Korennykh *et al.,* 2006). Ageritin has a pI of 8.6 that makes it a positively charged molecule in most of the intra- and extracellular matrix to facilitate cell entry and interaction with ribosomes. In contrast, its paralog has a pI value of 5.8, and the ineffectiveness of the paralog towards *Ae. aegypti* could be explained by its unfavorable electrostatic interactions with the cell membrane and ribosomes.

Ribotoxins cleave the ribosomal RNA in the universally conserved GAGA tetra loop found in the sarcin-ricin loop of the large ribosomal subunit. Therefore, all known ribosomes are potentially susceptible and a ribotoxin producing fungus has to avoid self-intoxication. Protection can be achieved by preventing the active ribotoxin to get in touch with ribosomes by compartmentalization and efficient secretion systems. However, unlike all previously described ribotoxins, ageritin has no known signal peptide sequence and is thus either retained in the cytoplasm or secreted using a yet unknown unconventional secretion pathway in *A*. *aegerita.*

We could express ageritin in a highly soluble manner in the cytoplasm of *E. coli.*

The high toxicity of ageritin against mosquito larvae shows that ageritin is highly suitable as a new bio-insecticide. Novel mosquito control agents are needed since insecticide resistance to currently available insecticides is a key challenge and more and more insecticides are phased out. Protective interventions such as insecticide-treated bed nets and indoor residual spraying become more and more ineffective. In addition, the increasing spread of invasive *Aedes* mosquitoes leads to emergence of arboviral diseases such as dengue and chikungunya in more temperate regions (Barzon, 2018). For example, *Ae. albopictus* has invaded large parts of southern Europe. This insect is an efficient vector viral diseases of dengue, chikungunya and zika virus (Kampen *et al.,* 2017).

### Further preferred embodiments

Ageritin, a ribotoxin, is used for fungal self-protection against parasites. Outside the fungal host, Ageritin has shown dosage dependent insecticidal activity towards mosquito larvae of the species *Aedes aegypti,* an important vector of tropical diseases, which is not indigenous in Europe with the exception of Madeira and Georgia. Therefore, a direct close relationship between the fungi and the mosquito can be excluded.

Ageritin, since produced naturally by a fungus, can be considered as a bioinsecticide. New insecticides are urgently needed since regulatory authorities are taking more and more chemical synthetic insecticides from the market due to safety reason.

### Pest insects presenting a target for Ageritin

### - Formulation of an insecticidal agent

The mode in which an insecticide is presented to pest insects is crucial. The active substance has to be brought in contact with the site of action within the insect. Most of the chemical insecticides have a major advantage since they pass the epidermis due to their lipophilic properties. Microbial insecticides such as *Bacillus thuringiensis* have to be ingested by the insects in order to display their insecticidal activity. Ageritin, presented within *E. coli* to mosquito larvae is also considered as microbial insecticide. Thus, insecticides, which have to be ingested, need to be formulated and applied in such a way that they are taken up by the target insects in high enough dosages.

A preferred embodiment is the transformation of culture plants with genes expressing insecticides. This technique is presently confined to genes expressing toxins of *B. thuringiensis*, and used against pest insects, which ravage in the inside of culture crops. Depending on the specificity of Ageritin, plant protection can be extended to pest insects feeding on the outside, e.g. on leaves and roots.

### - Expression of Ageritin in culture plants

For the development of GM-plants (genetically modified), the same approach can be used as in the case of cry-genes (transformation of plants with *B. thuringiensis* genes expressing insecticidal proteins in plants).

In 2018, the total global acreage of biotech crops amounted to 190 mio acres. Table 1 presents GMO-crops used in the field for the protection against pest insects. They all contain cry-genes *of B. thuringiensis.*

**Table 1. Transformation of major culture crops with the Ageritin gene for protection against pest insects.**

| **Target insects** | **Crops** | **Significance/Remarks** |
|---|---|---|
| ***Lepidoptera such as*** | | |
| *Ostrinia nubilalis* (European corn borer) | corn (maize) | Bt-corn is world-wide established. In the US 90% of the corn crop is based on Bt-corn. Ageritin-corn can be used for: |
| | | - live-stock feed, |
| | | - sweeteners, such as corn-syrup, |
| | | - fuel production, |
| | | - industrial uses, |
| *Spodoptera exigua S. frugiperda, Pectinophora gossypiella, Heliothis virescens Helicoverpa zea* | cotton | Fiber |
| | | Animal feed |
| | | Cotton seed-oil |
| *Chrysodeixis includes Helicoverpa zea, Helicoverpa armigera* | soybeans | Cattle feed, vegetable oil, tofu and retail food products. |
| Cutworms, various species, including: *Agrotis* spp. *Peridroma saucia Nephelodes minians* | brinjal (aubergine) | Important staple vegetable in South-East Asia |
| Hornworms: *Manduca quinquemaculata Manduca sexta* | | |
| Lepidopteran complex (e.g. rice stem borer) | rice | Approved by the Chinese Ministry of Agriculture in 2009 |

| ***Coleoptera such as*** | | |
|---|---|---|
| Colorado potato beetle (*Leptinotarsa decemlineata*) | potatoes, | |
| | corn, | |
| | soybeans | |

| ***Sucking insects such as*** | | |
|---|---|---|
| *Thrips* (Thysanoptera) | corn, | |
| Aphids (Hemiptera) | cotton, | |
| white flies (Homoptera) | brinjal | |

### - Ageritin for use as a conventional insecticide in plant protection in different formulations

Large-scale production of Ageritin for field application: It has been shown that Ageritin can be produced in cells of *E. coli.* Ageritin can be produced under controlled conditions in bioreactors, similar to the manufacturing of *Bacillus thuringiensis.* Following the growth phase, *E. coli* cells are harvested either by centrifugation and/or by a spray-drying process. The dried *E. coli* cells retain their insecticidal activity. The concentrated or dried cell material is formulated depending on the designated use as either wettable powders, liquid concentrates, granules or baits.

**Table 2 Ageritin for use as conventional insecticide in crop protection**

| **Organism** | **Crop** | **Formulation** |
|---|---|---|
| Slugs | Arable farming | Baits |
| Seed treatment | Arable farming | |
| Blossom beetle Rape stem weevil | Canola | Sprays |
| Aphids | Different crops | Sprays |
| *Drosophila susukii* | Soft fruits, grapes | Sprays |
| Raspberry weevil | Raspberries and other fruits and plants | Baits |
| Diptera Caterpillars | Many plants, especially vegetables | Sprays |
| Fungus gnats | Indoor plants, indoor-farmed edible mushrooms, e.g. *Agaricus bisporus* (button mushroom) | Liquids |
| European grape vine moth | Vine | Liquids |

### - Ageritin as an insecticide in public health and hygiene

Novel insecticides in the sectors of public health and hygiene are in urgent need. The emphasis in public health lies on the control of mosquitoes representing vectors of human infectious diseases. The main vectors are listed in Table 3a. It has to be added that the approach with Ageritin is of special interest since we have clearly demonstrated its mosquitocidal activity.

**Table 3a Use of Ageritin in public health, the control of mosquito vectors of human diseases**

| **Public Health** | |
|---|---|
| **Mosquitoes** | **Diseases** |
| ***Aedes* spp.** | |
| *Aedes aegypti* | Dengue, Chikungunya, Zika Yellow fever |
| *Aedes albopictus* | Dengue, Chikungunya, Zika |

| ***Culex* spp.** | |
|---|---|
| *Culex pipiensis* complex | West Nile fever |
| *Culex quinquefasciatus* | Lymphatic filariasis |
| | |

| ***Anopheles* spp.** | |
|---|---|
| *Anopheles gambiae, An. funestus, An. arabiensis* | Malaria |

**Table 3b. Use of Ageritin for the control of insects causing hygienic problems**

| **Hygiene** | **Formulation** |
|---|---|
| Cockroaches | Baits |
| Ants, termites | Baits |
| Bed bugs (these insects are of increasing importance) | *Beauveria bassiana* transformed with the gene expressing Ageritin |

The following examples and drawings illustrate the present invention without, however, limiting the same thereto.

### BRIEF DESCRIPTION OF THE DRAWINGS

**Figure 1****.** *Expression pattern and in vitro rRNA cleavage activity of ageritin.*
   **A)** Amino acid sequences of ageritin and its paralog were aligned using the ClustalW algorithm (v2.1). Blue shades indicate conservation of the residues.
   **B)** Expression level for the ageritin-encoding gene *AaeAGT1* and the paralog *AaeAGT2* at different stages of fruiting body development relative to that of vegetative mycelium (developmental stage I). The dotted horizontal line represents the mycelial expression level of both *AaeAGT1* and *AaeAGT2* and is used as a base-line value (developmental stage I), in comparison with other developmental stages of *A. aegerita* AAE-3: II, fruiting-primed mycelium 24 h to 48 h before emergence of visible fruiting body (FB) initials; III, FB initials; IV, entire FB primordia; Vs, young FB stipe; Vc, young FB cap. The error bars represent the standard deviation of three biological replicates.
   **C)** Ribonucleolytic activity of ageritin assayed with 400 nM of purified ageritin against ribosomes of rabbit reticulocyte lysate. Ribotoxin α-Sarcin and PBS buffer were used as positive and negative control, respectively. Ribosomal RNAs and classical ribotoxin cleavage product, α-fragment, were indicated.
**Figure 2**. *Toxicity of ageritin against mosquito larvae and Sf21 cells.*
   Toxicity of ageritin and its tagged version were tested against *Aedes aegypti* larvae by recording the mortality **(A)** and by the consumption *of E. coli* measured by the change of the OD₆₀₀ of a respective *E. coli* suspension **(B).** Mosquito larvae were feeding for 96 h on IPTG-induced *E. coli* BL21 expressing proteins Cgl2, Ageritin and His8-Ageritin. *E. coli* BL21 cells expressing either previously characterized insecticidal protein Cgl2 or carrying 'empty' vector (EV) were used as positive and negative control, respectively. Statistical analysis was done using Dunnett's multiple comparisons test. The error bars represent the standard deviation of five biological replicates. ******p* < 0.0001 vs. EV.
   **C)** Ageritin toxicity was tested against insect Sf21 cell lines. Different concentrations of purified ageritin were incubated with Sf21 cells for 72 h. The number of viable cells was counted for each sample. DMSO and ribotoxin α-Sarcin were used as positive controls, and PBS buffer was used as a negative control. Dunnett's multiple comparisons test was used for statistical analysis. The error bars represent the standard deviation of six biological replicates. Symbols/abbreviations: ns: not significant, ****p* < 0.001, ******p* < 0.0001 vs. PBS.
**Figure 3****.** *Effect of mutations in conserved residues on in vivo and in vitro activities of ageritin.*
   **A)** The amino acid sequence of ageritin was searched against JGI MycoCosm fungal database. The hit regions of the top 10 sequences with highest homology were aligned using the ClustalW algorithm (v2.1). Grey shading indicates the degree of conservation from dark to light. Individually mutated conserved regions are indicated by boxes and asterisks.
   **B)-D)** Insecticidal activity of mutated ageritin versions was monitored by feeding *Ae. aegypti* larvae with *E. coli* BL21 cells expressing proteins of interest. Wild type and mutated ageritin toxicity was assessed by counting the number of surviving larvae **(B),** measuring OD₆₀₀-based bacteria consumption **(C)** every day for four days and counting number of larvae that reached the adult stage **(D)** by the end of day 7 of the larvae feeding on bacteria expressing one of the corresponding proteins. *E.coli* BL21 expressing either previously characterized insecticidal protein Cgl2 or carrying 'empty' vector (EV) were used as positive and negative controls, respectively. Statistical analysis was done using Dunnett's multiple comparisons test. The error bars represent the standard deviation of five biological replicates. ******p* < 0.0001 vs. EV
   **E)** Ribonucleolytic activity of ageritin mutants was assessed with 400 nM of purified proteins using ribosomes of rabbit reticulocyte lysate. α-Sarcin and PBS buffer were used as positive and negative control, respectively. Ribosomal RNAs and ribotoxin cleavage product, α-fragment, were indicated.
**Figure 4****.** *Functional comparison between ageritin and its paralog.*
   **A)** Heterologous expression and solubility of ageritin paralog in *E. coli* BL21. 20 µl of either bacterial whole cell extract (WCE), supernatants of WCE after low speed spin (LS; 5 min at 5000 × g) or high speed spin (HS; 30 min at 16000 × g) were loaded on a SDS-PAGE and stained with Coomassie brilliant blue.
   **B)** Insecticidal activity of the ageritin paralog along with ageritin was tested against *Ae. aegypti* larvae by feeding the L3 staged mosquito larvae with *E. coli* BL21 expressing proteins of interests.
   Nematotoxicity of the ageritin paralog along with ageritin was assessed against *Caenorhabditis elegans* **(C)** *Caenorhabditis briggsae* **(D)** and *Distolabrellus veechi* **(E)** by counting number of developed larvae after two days of feeding on IPTG-induced *E. coli* BL21 expressing either previously characterized toxic protein Cgl2 or carrying 'empty' vector (EV) were used as positive and negative control, respectively. Dunnett's multiple comparisons test was used for statistical analysis. The error bars represent the standard deviation of three biological replicates. Symbols/abbreviations: ns: not significant, **p* < 0.05, **p* < *0.01,* ****p* < 0.001, ******p* < 0.0001 vs. EV.
**Figure 5****.** *Rooted circular cladogram of putative ageritin homologs.*
   Amino acid sequences of ageritin were searched against JGI MycoCosm fungal database. The complete amino acid sequence of top 30 hits were aligned using the ClustalW (v2.1), and phylogenetic relationships among the sequences were depicted via a circular cladogram. The hit with the lowest homology to ageritin among those 30 hits had an E-value of 7.2E⁻¹⁶ , an identity of 44.8% and a subject coverage of 64.8%. The branch lengths are relative and not to scale. Different potential ageritin homologs in the genome of a given species are labeled by numbers.
**Figure 6****.** *Expression levels of the reference genes for quantitative real-time reverse transcription-PCR.*
   *AaeIMP1* (gene ID AAE3_02268), *AaeTIF1* (gene ID AAE3_07769) and *AaeARP1* (gene ID AAE3_11594) from the genome sequence of the dikaryon *A. aegerita* AAE-3 (Gupta *et al.,* 2018), showing their transcription level by means of their Cq-values derived from quantitative real-time PCR analysis in a box-and-whisker-plot diagram. Whiskers indicate the variability outside the upper and lower quartile, respectively.
**Figure 7****.** *Assessment of expression and solubility of ageritin in E. coli.*
   **A)** Heterologous expression and solubility of ageritin and its His8-tagged version in *E. coli* BL21 cells. 20 µl of whole cell extract (WCE), supernatants of low speed spin (LS; 5 min. at 5000 × g) and high speed spin (HS; 30 min. at 16000 × g) of bacterial lysate were loaded on SDS-PAGE gel and stained with Coomassie brilliant blue. Cg12 was used as a positive control for IPTG-induced expression and solubility analysis.
   **B)** His8-ageritin was produced in *E. coli* BL21 and 20 µl of Ni-NTA purified protein loaded onto the SDS-PAGE along with 20 µl of WCE and stained with Coomassie brilliant blue.
   **C)** Ageritin single-site mutant constructs were produced in *E. coli* BL21, 20 µL of WCE, LS and HS of each bacterial lysate were loaded onto an SDS-PAGE and stained with Coomassie brilliant blue. **D)** 5 µl of purified protein (P) of each mutant ageritin version were loaded on a SDS-PAGE along with 20 µl of its WCE and stained with Coomassie brilliant blue.
**Figure 8****.** *Ageritin nematotoxicity tests.*
   Potential nematotoxicity of ageritin was tested against five different bacterivorous nematodes species: *Caenorhadbitis elegans*, *C. briggsae*, *C. tropicalis*, *Distolabrellus veechi* and *Pristionchus pacificus.* IPTG-induced *E. coli* BL21 expressing previously characterized nematotoxic protein Cgl2 or carrying 'empty' vector (EV) were used as positive and negative control, respectively. Dunnett's multiple comparisons test was used for statistical analysis. The error bars represent the standard deviation of three biological replicates. Symbols/abbreviations: ns: not significant, **p* < 0.05, ***p* < 0.01, ****p* < 0.001, ******p* < 0.0001 vs. EV

### EXAMPLES

### EXAMPLE

### Materials and Methods

### 1. Strains and cultivation conditions

Cultivation and strain maintenance of *A. aegerita* AAE-3 was performed as described previously (Herzog *et al.,* 2016). *Escherichia coli* strains DH5α and BL21 (DE3) were used for cloning and protein expression, respectively. Other organisms including microorganisms used in this study are listed in Table 4.

**Table 4. Organisms used in this study.**

| **Name** | **Strain** | **Source/Reference** |
|---|---|---|
| *Caenorhabditis briggsae* | AF16 | Caenorhabditis Genetics Center (CGC) |
| *Caenorhabditis elegans* | N2 | Caenorhabditis Genetics Center (CGC) |
| *Caenorhabditis tropicalis* | JU1373 | Caenorhabditis Genetics Center (CGC) |
| *Distolabrellus veechi* | environmental isolate | Luis Lugones, Utrecht University, Netherlands |
| *Pristionchus pacificus* | PS312 | lain Wilson, BOKU, Vienna, Austria |
| *Aedes aegypti* | Rockefeller | Pie Muller, Swiss Tropical and Public Health Institute, Basel, Switzerland |
| *Agrocybe aegerita* | AAE-3 | Florian Hennicke, Senckenberg BiK-F, Frankfurt a.M., Germany; genome-sequenced (Gupta *et al.,* 2018) |
| *Escherichia coli* | DH5α | |
| *Escherichia coli* | BL21(DE3) | Novagen |

### 2. Isolation of total RNA from A. aegerita for assessing expression of AaeAGT1 and AaeAGT2

Fruiting induction for the sake of sampling different stages of fruiting body development of *A*. *aegerita* AAE-3 was performed as described by Herzog *et al.* (2016). In brief, 1.5% w/v malt extract agar (MEA) plates were inoculated centrally with a 0.5 cm diameter agar plug originating from the growing edge of an *A. aegerita* AAE-3 culture. Before fruiting induction (20 °C, 12 h/12 h light/dark cycle, saturated humidity, aeration, local injury of the mycelium by punching out a 0.5 cm diameter mycelium-overgrown MEA plug), fungal plates were incubated for ten days at 25 °C in the dark.

Samples, consisting of at least three independent replicates, were retrieved from the following developmental stages: I) vegetative mycelium prior to fruiting induction at day ten post inoculation; II) fruiting-primed mycelium 24 h to 48 h before emergence of fruiting body initials at day 14 post inoculation; III) fruiting body initials at day 15 to 16 post inoculation; IV) fruiting body primordia at day 17 to the morning of day 19 post inoculation; Vs-Vc) young fruiting bodies separated into stipe (Vs) and cap (Vc) plectenchyme at day 19 to the morning of day 21 post inoculation. Mature fruiting bodies exhibiting full cap expansion and a spore print emerging by morning of day 22 post inoculation were not sampled. Sample I) and II) were obtained by gently scraping off the outermost 1 cm of mycelium from three replicate agar plates by gently scraping with a sterile spatula. Samples were transferred immediately to a 2 mL microcentrifuge tube containing 1 mL of RNAlater® (product ID: R0901, Sigma Aldrich GmbH, Munich, Germany) which was transferred to 4 °C for a maximum of 3 days before freezing at -80 °C until total RNA extraction.

For total RNA extraction, NucleoSpin® RNA Plant kit (product ID: 740949, Macherey-Nagel GmbH & Co. KG, Düren, Germany) was used whereby cell homogenization and lysis were modified. First, the RNAlater® was removed from each pooled sample and an appropriate amount of lysis Buffer RA1 added (350 µl per 100 mg fungal biomass). One 4 mm- and about ten 1 mm-diameter acetone-cleaned stainless-steel beads (product IDs: G40 and G10, respectively, KRS-Trading GmbH, Barchfeld-Immelborn, Germany) were then added to each tube. Homogenization was achieved using a mixer mill MM 200 (Retsch, Haan, Germany) set to 8 min at 25 Hz. Then, the protocol followed the recommendations of the manufacturer for RNA-extraction from filamentous fungi, including a DNA digestion step with the kit's rDNase, beginning with the "filtrate lysate" step.

Total RNA extracts were eluted in nuclease-free water (product ID: T143, Carl Roth GmbH & Co. KG, Karlsruhe, Germany) and the RNA concentration was measured spectrophotometrically with a NanoDrop 2000c (Thermo Fisher Scientific, Waltham, USA). RNA quality was visually assessed by checking the integrity of the major rRNA bands in a Urea-PAGE. Per lane, 1 µg of total RNA was loaded onto pre-cast 6% TBE-Urea polyacrylamide gels (product ID: EC68652BOX, Thermo Fischer Scientific, Waltham, USA) and separated for 1 h at a constant voltage of 180V. For the detection SYBR™ Gold (product ID: S11494, Thermo Fisher Scientific) was used to stain the gel following the manufacturer's recommendations. Only if no degradation of the RNA was observed, with major rRNA bands intact, the respective sample was further processed. Total RNAs were routinely stored at -80 °C.

### 3. Determination of suitable reference genes for quantitative real-time reverse transcription-PCR

Primer pairs for *A. aegerita* AAE-3 genes *AaeIMP1* (gene ID AAE3_02268), *AaeTIF1* (gene ID AAE3_07769) and *AaeARP1* (gene ID AAE3_11594) have been designed manually on the basis of their genomic DNA (Gupta *et al*., 2018). In each case, the gene name was assigned in accordance with the name of the putative encoded protein according to the UniProt database (www.uniprot.org). Each primer pair (Table 5, see below) spans a cDNA nucleotide region of 150 bp. Twelve reference samples were taken from vegetative mycelia grown for 10, 14, 18, 20, 22, 24 and 27 days on agar plates, from a primordia source of 18 day old cultures, and from fruiting bodies before (two samplings on different days from young fruiting bodies), during (one sampling) and after sporulation (one sampling). The samples were stored in RNAlater (Qiagen, Venlo, Netherlands). Total RNA was extracted using TRIzol (Thermo Fischer Scientific) by the method of Chomczynski and Sacchi (1987). The RNA concentration was determined by the absorbance at 260 nm using a Pearl Nanophotometer (Implen, Munich, Germany). 2 µg total RNA of each sample was reverse transcribed applying the M-MLV reverse transcriptase kit according to the manufacturer's protocol (Thermo Fischer Scientific) using oligo-(dT)₃₀-Primer. The resulting cDNA sample was incubated for 20 min at 37 °C with 1 µL AMRESCO RNase A (VWR International, Radnor, PA, USA) instead of the RNase H described in the M-MLV reverse transcriptase kit protocol. Quantitative real-time polymerase chain reaction was conducted in triplicates using the KAPA SYBR® FAST Universal Kit (Sigma Aldrich GmbH) according to the manufacturer's protocol with an annealing step at 60 °C for 30 sec, an elongation step at 72 °C for 10 sec in a total volume of 25 µL with a final concentration of 0.9 µM for each primer and 7 µL of cDNA template on a CFX connect Real-Time Detection System (Bio-Rad, Hercules, CA, USA).

To test primer efficiency, cDNA samples were combined in equal parts. Six logarithmic dilution steps of the cDNA master mix were used for qPCR (see above). Cq-values of the dilutions were blotted versus the dilution factor and linear regression as well as the corresponding determination coefficient was calculated for each reference gene. The primer efficiency was calculated according to Pfaffl (2001). For validation of the reference genes, all 12 cDNA samples were used separately for a quantitative real-time PCR analysis with all three primer pairs. Cq-values were used for validation using the NormFinder (Andersen *et al.,* 2004) and geNorm (Vandesompele *et al.,* 2002) algorithm.

### 4. One-step quantitative real-time reverse transcription-PCR (qRT-PCR) using total RNA samples

Primers and qRT-PCR conditions were designed according to the general recommendations of the MIQE guidelines (Bustin *et al.,* 2009). The software Geneious R11 (https://www.geneious.com, Kearse *et al.* (2012) was applied to design the primers for the genes encoding ageritin (*AaeAGT1*, gene ID AAE3_01767) and its paralog (*AaeAGT2*, gene ID AAE3_01768) as well as the two reference genes *AaeTIF1* (gene ID AAE3_07669) and *AaeIMP1* (gene ID AAE3_02268) from the *A. aegerita* AAE-3 genome sequence (Gupta *et al.,* 2018; see also www.thines-lab.senckenberg.de/agrocybe_genome). Primers for these genes are also listed in Table 5.

All qRT-PCRs were performed on an AriaMX Real-Time PCR System (Agilent Technologies, La Jolla, CA, USA) in optically clear 96-well plates with 8-cap strips using the Brilliant Ultra-Fast SYBR Green QRT-PCR Master Mix (Agilent Technologies). This one-step master mix included the reverse transcriptase (RT), the reaction buffer, the DNA polymerase and SYBR green. The RT-reaction was performed in each well prior to the start of the qRT-PCR program. All samples were run in three biological replicates with 25 ng total RNA per reaction as template. Constant qRT-PCR reaction components across the same-amplicon-PCRs were prepared as master mixes. The final concentration per primer was 250 nM. A melting curve analysis was done for each reaction at the end of the qRT-PCR to determine amplicon purity. See Table 3 for the qRT-PCR program.

**Table 5. Primers used in this study.**

| **Primer^{a}** | **Sequence 5' - 3^{'b}** | SEQ ID NO. |
|---|---|---|
| pAGT1-Nd | ggcgcatATGTCCGAGTCCTCTACCTTCACCACTGC | 5 |
| pAGT1-N | gtgcggccgcTCACGCCGGAGCCTTGCCC | 6 |
| pF_8His-Ag | CACCACCACCACGAGTCCTCTACCTTCACCACTG | 7 |
| pR_8His-Ag | ATGATGATGATGGGACATATGTATATCTCCTTCT | 8 |
| pF_8His-Ag(Y57A) | AAGTTGGTCACGGCCACCAGCCGCC | 9 |
| pR_8His-Ag(Y57A) | GGTCTTATCAATTTTGTCCTTCCCCG | 10 |
| pF_8His-Ag(R87A) | GTCGCGCTCGACATGGACAACACC | 11 |
| pR_8His-Ag(R87A) | GTAGGGCACGATGGAGCCCGCGGC | 12 |
| pF_8His-Ag(D89A) | TGCCCTACGTCCGGCTCGCCATGG | 13 |
| pR_8His-Ag(D89A) | CGATGGAGCCCGCGGCCGTTTTGA | 14 |
| pF_8His-Ag(D91A) | GTCCGGCTCGACATGGCCAACACC | 15 |
| pR_8His-Ag(D91A) | GTAGGGCACGATGGAGCCCGCGGC | 16 |
| pF_8His-Ag(H98A) | GGCAAGGGCATCGCTTTCAA | 17 |
| pR_8His-Ag(H98A) | GGTGTTGTCCATGTCGAGCC | 18 |
| pF_8His-Ag(K110A) | AGTTCCGCCGCGCTCGCCG | 19 |
| pR_8His-Ag(K110A) | GTCGGAGAGTTTAGTCGCGTTGAAA | 20 |
| cds01767-f | TTCTTTTCGCTACTCAGAATCGTTG | 21 |
| cds01767-r | CAGAGCTCTCCCAACCACAG | 22 |
| 02268_f | AGATGCGTATTCTGATGGTTGGTC | 23 |
| 02268_r | CCCACACTGTGAATGAGATGTTC | 24 |
| 07769_f | ATTCCTACGATCCTTTTGCCG | 25 |
| 07769_r | GATCATATTGTTTCGGGAGTCCT | 26 |
| 11594_f | TCTGATCTGACTGTCGGCCAA | 27 |
| 11594_r | ATCCTCGTCCTTATGCTCCTC | 28 |
| 01767_f | AAGCCCCGCATATCAGAAG | 29 |
| 01767_r | CTGTCGGAGAGTTTAGTCGC | 30 |
| 01768_f | GAAAGACCCAGATTGACCCAG | 31 |
| 01768_f | GTGAATTTTAGGCCGACGC | 32 |

| | | |
|---|---|---|
| ^{a}Primers used for quantitative real-time PCR procedures are labelled with forward (f) and reverse (r), respectively. Primers for cDNA generation start by coding sequence (cds) and end by forward (fw) or reverse (rv). ^{b}Lowercase letters are primer extensions to create the underlined restriction sites. | | |

**Table 6. qRT-PCR program for measuring AaeAGT1 and AaeAGT2 expression in this study.**

| **Programme step** | | **Temperature** | **Time** |
|---|---|---|---|
| Reverse transcription | | 50 °C | 10 min |
| Initial denaturation (hot start) | | 95 °C | 3 min |
| 40 cycles | Denaturation | 95 °C | 5 sec |
| | Annealing | 58 °C | 10 sec |
| | Extension | 72 °C | 10 sec |
| Melting curve | | 65 °C to 95 °C (resolution 0.5 °C) | 5 min |

### 5. Relative differential expression analysis

For data analysis the fluorescence readings were extracted from the raw run plots. The baseline correction and determination of the quantification cycle (Cq) and mean PCR efficiency (E) per amplicon was done according to Ruijter *et al.* (2009) using the LinRegPCR program in version 2017.1.

Relative expression ratios were calculated using the software REST, version REST2009. It employs the 'Pfaffl method' (Pfaffl, 2001) to calculate the E corrected relative gene expressions ratios allowing simultaneous the use of multiple reference genes for normalization based on Vandesompele *et al.* (2002). 95% confidence intervals around the mean relative expression ratios were calculated on the basis of 2,000 iterations. Vegetative mycelium that was not induced for fruiting (developmental stage I) was chosen as calibration sample.

### 6. cDNA generation from A. aegerita RNA

The cDNA was synthetized from total RNA of fruiting-primed mycelium using the RevertAid first strand cDNA synthesis kit (product ID: K1621, Thermo Fisher Scientific) and an oligo(dT)₁₈ primer. The first strand total-cDNA was then directly used as a template to produce specific cDNA of the ageritin-encoding gene *AaeAGT1* (gene ID AAE3_01767) with primer pair cds01767-f and cds01767-r (see Table 5) in a standard 3-step PCR using Phusion polymerase (product ID: F530, Thermo Fischer Scientific) with a annealing temperature of 62 °C . DNA sequence information for primer design was obtained from the genome sequence of *A. aegerita* AAE-3 (Gupta *et al*., 2018).
SEQ ID NO. 2 verified cDNA sequence of gene *AaeAGT1*, gene ID AAE3_01767:

atgtccgagtcctctaccttcaccactgcggtagtacctgaaggcgaaggagttgctccaatggcaga
gaccgtgcagtattacaactcctactctgacgcatccatcgcgtcttgcgcatttgtagactcgggga
aggacaaaattgataagaccaagttggtcacgtacaccagccgcctcgccgcaagccccgcatatcag
aaggtcgtcggcgtcggcctcaaaacggccgcgggctccatcgtgccctacgtccggctcgacatgga
caacaccggcaagggcatccatttcaacgcgactaaactctccgacagttccgccaagctcgccgcgg
tgctcaagacgacggtgtccatgaccgaggcacagcgaactcaactctacatggagtatatcaagggc
atcgagaatcggagtgcgcagtttatttgggactggtggaggacgggcaaggctccggcgtga

### 7. Construction of ageritin expression vectors

The coding sequence of ageritin was identified by BLASTing the published 25 N-terminal residues of ageritin against the predicted proteome of *Agrocybe aegerita* (Gupta *et al.,* 2018). The sequence was amplified with the primer pair pAGT1-Nd and pAGT1-N (see Table 5) from the *AaeAGT1-*cDNA and cloned into a pET-24b (+) expression vector. The sequence of the cloned cDNA was confirmed by DNA sequencing. The plasmid was transformed into *E*. *coli* BL21 cells. For expression of ageritin, BL21-transformants were pre-cultivated in Luria-Bertani (LB) medium supplemented with 50 mg/l kanamycin at 37 °C. At an OD600 of around 0.5, the cells were induced with 0.5 mM isopropyl β-D-1-thiogalactopyranoside (IPTG) (product ID: 18000, BioSynth AG, Switzerland) and cultivated over night at 16 °C. Expression and solubility of ageritin was checked as previously described (Künzler *et al.*, 2010).

### 8. Toxicity against mosquito larvae and nematodes

Egg masses of the yellow fever mosquito, *Aedes aegypti,* were harvested on filter papers from the Rockefeller laboratory colony reared at the Swiss Tropical and Public Health Institute (Basel, Switzerland). For the experiments, mosquito larvae were reared by placing 2 to 5 cm² small pieces of the egg paper, depending on the density of the eggs, into glass petri dishes containing tap water at 28 °C. The larvae hatched within a few hours. They were fed with finely ground commercially available food for ornamental fish.

The toxicity assays against the mosquito larvae were performed as described previously (Künzler *et al.,* 2010). In brief, larvae in their third stage (L3) were used for the toxicity assays. The food source was changed from fish food to *E*. *coli,* adjusted in all the bioassays to an OD₆₀₀ of 0.4. The mosquito larvae fed readily on *E*. *coli* and were able to develop to the adult stage. The consumption of empty vector *E*. *coli* by the mosquito larvae reduced the optical density. The bioassay was performed by transferring ten L3 larvae to Schott bottles containing 99 mL of tap water and 1 ml of *E. coli* cells expressing the desired. The mosquito larvae were kept in the dark at 28 °C and the toxicity was assessed by the larval mortality over the first four days and by the reduction in optical density. In addition, the number of adult mosquitoes, which were able to develop from the larvae, was counted after 7 days. The previously characterized entomo- and nematotoxic lectin Cgl2 was used as a positive control in all toxicity assays (Bleuler-Martinez *et al.*, 2011). Starvation controls were used to check whether the death of the larvae is due to toxicity or refrainment of the larvae from consuming the bacteria. Nematotoxicity assays against five different species of nematodes were performed as described before (Künzler *et al.*, 2010; Plaza *et al.*, 2016). Dunnett's multiple comparisons test was used to calculate the statistical differences between mean values of treatment and control groups.

### 9. Tagging and purification of ageritin

For the purification of recombinant ageritin over Ni-NTA columns (Macherey-Nagel), the protein was tagged with a polyhistidine(8)-tag at its N-terminus. A plasmid was constructed by PCR using pF_8His-Ag and pR_8His-Ag primer pair listed in Table 5. His8-ageritin was expressed as described for untagged ageritin. Protein purification was performed as described previously (Bleuler-Martinez *et al.,* 2011).

### 10. In vitro rRNA cleavage assay

To detect the rRNA cleavage activity, 20 µL of untreated rabbit reticulate lysate (product ID: L4151, Promega, Wisconsin, USA) was mixed with a final concentration of 400 nM ageritin or with the mutant versions in the reaction buffer (15 mM Tris-HCl, 15 mM NaCl, 50 mM KCl, 2.5 mM EDTA. pH 7.6) to a final volume of 30 µL (Kao *et al.,* 2001). The reaction mixture was incubated for one hour at 30 °C, and stopped by adding 3 µL of 10% SDS. RNAs were isolated from the reaction mixture by chloroform-phenol extraction. The RNAs were mixed with 2×RNA loading dye (Thermo Fisher Scientific) and denatured for 5 minutes at 65 °C, cooled on ice and run on 2% native agarose gel in cold TBE Buffer (Tris-borate-EDTA) for 30 minutes at 100 V. α-Sarcin (product ID: BCO-5005-1, Axxora, USA) and PBS buffer were used as positive and negative control, respectively.

### 11. Toxicity towards insect cells

Cytotoxicity of ageritin was tested against the insect cell line of *Spodoptera frugiperda* Sf21 (IPLB-Sf21-AE). The insect cells were pre-cultivated in Sf-900TM II SFM medium (Invitrogen, CA, USA) supplemented with streptomycin (100 µg/mL) and penicillin (100 µg/mL). Cells were diluted to a final density of 0.29 × 10⁶ /mL and 500 µL of per well were dispensed in 24-well plates. The insect cells were challenged with different concentrations of ageritin (0.1 µM, 1 µM and 10 µM) dissolved in PBS. The well plates were incubated for three days at 27 °C. The liquid medium was removed, and the cells were stained with 15 µl of 0.4% Trypan Blue solution. The number of alive unstained cells was determined under a microscope. α-Sarcin (Axxora) and 5% DMSO were used as positive controls whereas the PBS buffer served as a negative control. Dunnett's multiple comparisons test was used to test whether the observed differences between the mean values of the treatment and control groups are statistically significant.

### 12. Alignment and phylogenetic tree

The complete amino acid sequence of ageritin was BLASTed against the database of the Gene Catalog Proteins (GCP) at JGI MycoCosm (Grigoriev *et al.*, 2014). The hit regions of the top ten sequences with highest homology were aligned using the ClustalW algorithm (v2.1) at CLC Genomics Workbench (Chenna *et al*., 2003).

For the analysis of the phylogenetic relationship among the top homologs of ageritin, the complete amino acid sequences of 30 hits were aligned using ClustalW (v2.1). A phylogenetic tree was constructed employing the Maximum Likelihood algorithm (Aldrich, 1997). The tree was designed as a rooted circular cladogram. The hit with the lowest homology to ageritin among the 30 hits had an E-value of 7.2E-16.

### 13. Creation and expression of mutant versions

Based on the alignment, six of the completely conserved residues (Y57, R87, D89, D91, H98 and K110) of ageritin were mutated individually to alanine using the site-specific primers listed in Table 5. The plasmid encoding His8-ageritin was used as template for construction of single-site mutants. Expression and purification of the mutant ageritin variants were done as for wild type His8-ageritin.

### 14. Expression of ageritin paralog

The coding sequence for the paralog of ageritin (63% sequence identity) was ordered from GenScript (Piscataway, NJ, USA). Expression and purification of the paralogous protein was done as described above for ageritin.

The features disclosed in the foregoing description, in the claims and/or in the accompanying drawings may, both separately and in any combination thereof, be material for realizing the invention in diverse forms thereof.

### REFERENCES

Aldrich J: R. A. Fisher and the making of maximum likelihood 1912-1922. Stat Sci 1997, 12(3):162-176.
Andersen CL, Jensen JL, Orntoft TF: Normalization of real-time quantitative reverse transcription-PCR data: A model-based variance estimation approach to identify genes suited for normalization, applied to bladder and colon cancer data sets. Cancer Res 2004, 64(15):5245-5250.
Barzon L. Ongoing and emerging arbovirus threats in Europe. J Clin Virol. 2018 Oct;107:38-47. doi: 10.1016/j.jcv.2018.08.007.
Berry et al. Appl Environ Microbiol. 2002;68:5082-95.
Bleuler-Martinez S, Butschi A, Garbani M, Walti MA, Wohlschlager T, Potthoff E, Sabotic J, Pohleven J, Luthy P, Hengartner MO et al: A lectin-mediated resistance of higher fungi against predators and parasites. Mol Ecol 2011, 20(14):3056-3070.
Bolognesi A, Bortolotti M, Maiello S, Battelli MG, Polito L: Ribosome-Inactivating Proteins from Plants: A Historical Overview. Molecules 2016, 21(12).
Bustin SA, Benes V, Garson JA, Hellemans J, Huggett J, Kubista M, Mueller R, Nolan T, Pfaffl MW, Shipley GL et al: The MIQE Guidelines: Minimum Information for Publication of Quantitative Real-Time PCR Experiments. Clin Chem 2009, 55(4):611-622.
Cantón PE, Zanicthe Reyes EZ, Ruiz de Escudero I, Bravo A, Soberón M. Binding of Bacillus thuringiensis subsp. israelensis Cry4Ba to CytlAa has an important role in synergism. Peptides. 2011 Mar;32(3):595-600. doi: 10.1016/j.peptides.2010.06.005. Epub 2010 Jun 15.
Cadavid-Restrepo G, Sahaza J, Orduz S. Treatment of an Aedes aegypti colony with the Cry11Aa toxin for 54 generations results in the development of resistance. Mem Inst Oswaldo Cruz. 2012 Feb;107(1):74-9.
Chan YL, Endo Y, Wool IG: The Sequence of the Nucleotides at the Alpha-Sarcin Cleavage Site in Rat 28-S Ribosomal Ribonucleic-Acid. J Biol Chem 1983, 258(21):2768-2770.
Cheng et al., 2004, J Agric Food Chem 52:4395-4400.
Cheng et al., 2009, Bioresour Technol. 100:457-464.
Chenna R, Sugawara H, Koike T, Lopez R, Gibson TJ, Higgins DG, Thompson JD: Multiple sequence alignment with the Clustal series of programs. Nucleic Acids Research 2003, 31(13):3497-3500.
Chomczynski P, Sacchi N: Single-Step Method of Rna Isolation by Acid Guanidinium Thiocyanate Phenol Chloroform Extraction. Anal Biochem 1987, 162(1):156-159.
Cloonan KR, Andreadis SS, Chen H, Jenkins NE, Baker TC. Attraction, Oviposition and Larval Survival of the Fungus Gnat, Lycoriella ingenua, on Fungal Species Isolated from Adults, Larvae, and Mushroom Compost. PLoS One. 2016 Dec 9;11(12):e0167074. doi: 10.1371/journal.pone.0167074. eCollection 2016.
Correll CC, Munishkin A, Chan YL, Ren Z, Wool IG, Steitz TA: Crystal structure of the ribosomal RNA domain essential for binding elongation factors. P Natl Acad Sci USA 1998, 95(23):13436-13441.
Endo Y, Tsurugi K, Yutsudo T, Takeda Y, Ogasawara T, Igarashi K: Site of action of a Vero toxin (VT2) from Escherichia coli O157:H7 and of Shiga toxin on eukaryotic ribosomes. RNA N-glycosidase activity of the toxins. Eur J Biochem 1988, 171(1-2):45-50.
Gupta DK, Rühl M, Mishra B, Kleofas V, Hofrichter M, Herzog R, Pecyna MJ, Sharma R, Kellner H, Hennicke F et al: The genome sequence of the commercially cultivated mushroom Agrocybe aegerita reveals a conserved repertoire of fruiting-related genes and a versatile suite of biopolymer-degrading enzymes. Bmc Genomics 2018, 19.
Grigoriev IV, Nikitin R, Haridas S, Kuo A, Ohm R, Otillar R, Riley R, Salamov A, Zhao XL, Korzeniewski F et al: MycoCosm portal: gearing up for 1000 fungal genomes. Nucleic Acids Research 2014, 42(D1):D699-D704.
Herrero-Galan E, Lacadena J, del Pozo AM, Boucias DG, Olmo N, Onaderra M, Gavilanes JG: The insecticidal protein hirsutellin A from the mite fungal pathogen Hirsutella thompsonii is a ribotoxin. Proteins 2008, 72(1):217-228.
Herrero-Galán E, Álvarez-García E, Carreras-Sangrà N, Lacadena J, Alegre-Cebollada J, Martínez del Pozo Á, Oñaderra M, Gavilanes JG: Fungal ribotoxins: structure, function and evolution. In: Microbial Toxins: Current Research and Future Trends. 2009: 167-187.
Herrero-Galán E, Garcia-Ortega L, Lacadena J, Martinez-Del-Pozo Á, Olmo N, Gavilanes JG, Oñaderra M: Implication of an Asp residue in the ribonucleolytic activity of hirsutellin A reveals new electrostatic interactions at the active site of ribotoxins. Biochimie 2012, 94:427-433.
Herrero-Galán E, Garcia-Ortega L, Olombrada M, Lacadena J, Del Pozo ÁM, Gavilanes JG, Oñaderra M: Hirsutellin A: A Paradigmatic Example of the Insecticidal Function of Fungal Ribotoxins. Insects 2013, 4:339-356.
Herzog R, Solovyeva I, Ruhl M, Thines M, Hennicke F: Dikaryotic fruiting body development in a single dikaryon of Agrocybe aegerita and the spectrum of monokaryotic fruiting types in its monokaryotic progeny. MycolProg 2016, 15(9):947-957.
Kampen H, Schuhbauer A, Walther D: Emerging mosquito species in Germany-a synopsis after 6 years of mosquito monitoring (2011-2016). Parasitol Res 2017, 116(12):3253-3263.
Kao R, Martinez-Ruiz A, Del Pozo AM, Crameri R, Davies J: Mitogillin and related fungal ribotoxins. Methods in Enzymology 2001, 341:324-335.
Kearse M, Moir R, Wilson A, Stones-Havas S, Cheung M, Sturrock S, Buxton S, Cooper A, Markowitz S, Duran C et al: Geneious Basic: An integrated and extendable desktop software platform for the organization and analysis of sequence data. Bioinformatics 2012, 28(12):1647-1649.
Korennykh AV, Piccirilli JA, Correll CC: The electrostatic character of the ribosomal surface enables extraordinarily rapid target location by ribotoxins. Nat Struct Mol Biol 2006, 13(5):436-443.
Künzler M, Bleuler-Martinez S, Butschi A, Garbani M, Lüthy P, Hengartner MO, Aebi M: Biotoxicity assays for fruiting body lectins and other cytoplasmic proteins. 2010, 480:141-150.
Lacadena J, del Pozo AM, Martinez-Ruiz A, Perez-Canadillas JM, Bruix M, Mancheno JM, Onaderra M, Gavilanes CG: Role of histidine-50, glutamic acid-96, and histidine-137 in the ribonucleolytic mechanism of the ribotoxin alpha-sarcin. Proteins-Structure Function and Genetics 1999, 37(3):474-484.
Lacadena J, Alvarez-Garcia E, Carreras-Sangra N, Herrero-Galan E, Alegre-Cebollada J, Garcia-Ortega L, Onaderra M, Gavilanes JG, del Pozo AM: Fungal ribotoxins: molecular dissection of a family of natural killers. Fems Microbiol Rev 2007, 31(2):212-237.
Landi N, Pacifico S, Ragucci S, Iglesias R, Piccolella S, Amici A, Di Giuseppe AMA, Di Maro A: Purification, characterization and cytotoxicity assessment of Ageritin: The first ribotoxin from the basidiomycete mushroom Agrocybe aegerita. Biochimica et Biophysica Acta - General Subjects 2017, 1861:1113-1121.
Olombrada M, Herrero-Galán E, Tello D, Oñaderra M, Gavilanes JG, Martinez-Del-Pozo Á, Garcia-Ortega L: Fungal extracellular ribotoxins as insecticidal agents. Insect Biochem Mol Biol 2013, 43 39-46.
Olombrada M, Martinez-Del-Pozo Á, Medina P, Budia F, Gavilanes JG, Garcia-Ortega L: Fungal ribotoxins: Natural protein-based weapons against insects. Toxicon 2014, 83:69-74.
Olombrada M, Medina P, Budia F, Gavilanes JG, Martinez-Del-Pozo A, Garcia-Ortega L: Characterization of a new toxin from the entomopathogenic fungus Metarhizium anisopliae: the ribotoxin anisoplin. Biol Chem 2017, 398(1):135-142.
Olombrada M, Lázaro-Gorines R, López-Rodríguez J, Martinez-del-Pozo Á, Oñaderra M, Maestro-Lopez M, Lacadena J, Gavilanes J, Garcia-Ortega L: Fungal Ribotoxins: A Review of Potential Biotechnological Applications. Toxins 2017, 9:71.
Plaza DF, Schmieder SS, Lipzen A, Lindquist E, Künzler M: Identification of a Novel Nematotoxic Protein by Challenging the Model Mushroom Coprinopsis cinerea with a Fungivorous Nematode. Genes|Genomes|Genetics 2016, 6:87-98.
Perez-Canadillas JM, Santoro J, Campos-Olivas R, Lacadena J, del Pozo AM, Gavilanes JG, Rico M, Bruix M: The highly refined solution structure of the cytotoxic ribonuclease alpha-sarcin reveals the structural requirements for substrate recognition and ribonucleolytic activity. Journal of Molecular Biology 2000, 299(4):1061-1073.
Pfaffl MW: A new mathematical model for relative quantification in real-time RT-PCR. Nucleic Acids Research 2001, 29(9).
Pfaffl MW, Horgan GW, Dempfle L: Relative expression software tool (REST (c)) for group-wise comparison and statistical analysis of relative expression results in real-time PCR. Nucleic Acids Research 2002, 30(9).
Ruijter JM, Ramakers C, Hoogaars WMH, Karlen Y, Bakker O, van den Hoff MJB, Moorman AFM: Amplification efficiency: linking baseline and bias in the analysis of quantitative PCR data. Nucleic Acids Research 2009, 37(6).
Sawa T, Hamaoka S, Kinoshita M, Kainuma A, Naito Y, Akiyama K, Kato H: Pseudomonas aeruginosa Type III Secretory Toxin ExoU and Its Predicted Homologs. Toxins 2016, 8(11).
Suter T, Crespo MM, de Oliveira MF, Alves de Oliveira MA, Varjal de Melo-Santos CM, Fontes de Oliveira CM, Junqueira Ayres CF, Rodrigues Barbosa RM, Araújo AP, Regis LN, Flacio E, Engeler L, Müller P, Neves Lobo Silva-Filha MH: Insecticide susceptibility of Aedes albopictus and Ae. aegypti from Brazil and the Swiss-Italian border region. Parasites & Vectors 2017, 10:431.
Vandesompele J, De Preter K, Pattyn F, Poppe B, Van Roy N, De Paepe A, Speleman F: Accurate normalization of real-time quantitative RT-PCR data by geometric averaging of multiple internal control genes. Genome Biology 2002, 3(7).
Weeks EN, Baniszewski J, Gezan SA, Allan SA, Cuda JP, Stevens BR. Methionine as a safe and effective novel biorational mosquito larvicide. Pest Manag Sci. 2018 Jun 11. doi: 10.1002/ps.5118.
Yang XJ, Moffat K: Insights into specificity of cleavage and mechanism of cell entry from the crystal structure of the highly specific Aspergillus ribotoxin, restrictocin. Structure 1996, 4(7):837-852.
Yao QZ, Yu MM, Ooi LSM, Ng TB, Chang ST, Sun SSM, Ooi VEC: Isolation and characterization of a type 1 ribosome-inactivating protein from fruiting bodies of the edible mushroom (Volvariella volvacea). J Agr Food Chem 1998, 46(2):788-792.

## Claims

1. A *protein* selected from
- a protein comprising an amino acid sequence of SEQ ID NO. 1 or an amino acid sequence having at least 60% sequence identity, preferably at least 70% sequence identity, more preferably at least 80% or 81% sequence identity to the amino acid sequence of SEQ ID NO. 1, and/or
- a protein encoded by a nucleotide sequence of SEQ ID NO. 2 or a nucleotide sequence having at least 60% sequence identity, preferably 70% sequence identity, more preferably 80% or 81% sequence identity to the nucleotide sequence of SEQ ID NO. 2.

2. The protein of claim 1, comprising an amino acid substitution in position Y57, D91 and/or K110.

3. A *nucleic acid molecule,* comprising a nucleotide sequence of SEQ ID NO. 2 or a nucleotide sequence having at least 60% sequence identity, preferably 70% sequence identity, more preferably 80% or 81% sequence identity to the nucleotide sequence of SEQ ID NO. 2, preferably coding for a protein according to claim 1 or 2.

4. The nucleic acid molecule of claim 3, further comprising vector nucleic acid sequences, preferably expression vector sequences,
and/or comprising promoter nucleic acid sequences and terminator nucleic acid sequences,
and/or comprising other regulatory nucleic acid sequences,
and/or wherein the nucleic acid molecule preferably comprises dsDNA, ssDNA, cDNA, LNA, PNA, CNA, RNA or mRNA or combinations thereof.

5. A *host cell,* containing a nucleic acid molecule according to claim 3 or 4 and preferably expressing said nucleic acid molecule,
and/or expressing the protein of claim 1 or 2.

6. The host cell of claim 5, which is selected from
- a bacterial cell,
preferably an *Escherichia* cell,
such as *Escherichia coli,*
- a plant cell,
preferably from an agricultural crop and/or an ornamental plant,
such as *Zea mays* (field corn: dent corn, flint corn, flour corn and blue corn; sweet corn: *Zea mays* convar. *saccharata* var. *rugosa*; popcorn: *Zea mays everta*)*, Gossypium* spp. (cotton species), *Capsicum* spp., *Solanum tuberosum, Solanum lycopersicum, Nicotiana tabacum, Phaseolus lunatus, Pisum sativum* var. *macrocarpon, Glycine max, Arachis hypogaea, Triticum aestivum, Avena sativa, Hordeum vulgare*, *Secale cereale, Malus domestica, Pyrus communis, Prunus* spp., e.g. *P. avium, P. persica, and P. domestica, Ribes* spp., e.g. *R. uva-crispa, R. nigrum,* and *R. rubrum, Vitis vinifera,*
or
- a fungal cell,
preferably from an edible, medicinal or ornamental (cultivated) mushroom or a yeast cell or an entomopathogenic or mite-pathogenic fungus,
such as *Agaricus bisporus, Pleurotus ostreatus, P. eryngii, Lentinula edodes, Hericium* spp., *Volvariella volvacea, Grifola frondosa, Ganoderma* spp., *Trametes* spp, *Auricularia polytricha*, *Flammulina velutipes, Lentinus (Pleurotus) sajor-caju, Hypsizygus tessellatus,*
*Ustilago* spp., e.g. *U. maydis, Microbotryum* spp., e.g. *M. lychnidis-dioicae,* and *M. violaceum, Xanthophyllomyces dendrorhous, Rhodotorula* spp., e.g. *R. glutinis, and R. mucilaginosa, Sporobolomyces* (*Sporidiobolus*) spp., *e.g. S. roseus, Mrakia* spp., e.g. *M. frigida, M. psychrophila,* and *M. gelida,*
*Beauveria* (*Cordyceps*) *bassiana, Hirsutella thompsonii, Isaria* (*Paecilomyces*) spp., e.g. *I. fumosorosea, Lecanicillium* spp., e.g. *L. longisporum, and L. muscarium, Metarhizium* spp., e.g. *M. anisopliae, and M. acridum, Nomuraea* spp., e.g. *N. rileyi.*

7. A recombinant protein of claim 1 or 2 obtained from a host cell of claim 5 or 6, wherein the host cell is preferably *Escherichia coli, Zea mays, Gossypium* spp. (cotton species), *Capsicum* spp., *Solanum tuberosum, Solanum lycopersicum, Nicotiana tabacum, Phaseolus lunatus, Pisum sativum* var. *macrocarpon, Glycine max, Arachis hypogaea, Triticum aestivum, Avena sativa, Hordeum vulgare*, *Secale cereale, Malus domestica, Pyrus communis, Prunus* spp., e.g. *P. avium, P. persica, and P. domestica, Ribes* spp., e.g. *R. uva-crispa, R. nigrum,* and *R. rubrum, Vitis vinifera.*

8. A *plant* or *fungus,*
containing a nucleic acid molecule according to claim 3 or 4 and preferably expressing said nucleic acid molecule,
and/or expressing the protein of claim 1 or 2,
and/or comprising host cell(s) of claim 5 or 6.

9. The plant or fungus of claim 8,
which is an agricultural crop and/or an ornamental plant,
such as *Zea mays* (field corn: dent corn, flint corn, flour corn and blue corn; sweet corn: *Zea mays* convar. *saccharata* var. *rugosa*; popcorn: *Zea mays everta*), *Gossypium* spp. (cotton species), *Capsicum* spp., *Solanum tuberosum, Solanum lycopersicum, Nicotiana tabacum, Phaseolus lunatus, Pisum sativum* var. *macrocarpon, Glycine max, Arachis hypogaea, Triticum aestivum, Avena sativa, Hordeum vulgare*, *Secale cereale, Malus domestica, Pyrus communis, Prunus* spp., e.g. *P. avium, P. persica, and P. domestica, Ribes* spp., e.g. *R. uva-crispa, R. nigrum,* and *R. rubrum, Vitis vinifera,*
or an edible, medicinal or ornamental (cultivated) mushroom,
such as *Agaricus bisporus, Pleurotus ostreatus, P. eryngii, Lentinula edodes, Hericium* spp., *Volvariella volvacea, Grifola frondosa, Ganoderma* spp., *Trametes* spp, *Auricularia polytricha, Flammulina velutipes, Lentinus (Pleurotus) sajor-caju, Hypsizygus tessellatus,*
or an entomopathogenic or mite-pathogenic fungus
such as *Beauveria* (*Cordyceps*) *bassiana, Hirsutella thompsonii, Isaria* (*Paecilomyces*) spp., e.g. *I. fumosorosea, Lecanicillium* spp., e.g. *L. longisporum, and L. muscarium, Metarhizium* spp., e.g. *M. anisopliae,* and *M. acridum, Nomuraea* spp., e.g. *N. rileyi.*

10. The plant of claim 8 or 9, furthermore comprising *Bacillus thuringiensis* or *Bacillus thuringiensis* subspecies *israelensis* (*Bti*)*.*

11. Use of
- the protein of claim 1, 2 or 7,
- the nucleic acid molecule of claim 3 or 4,
- the host cell of claim 5 or 6, and/or
- the plant or the fungus of any one of claims 8 to 10,
as (bio)insecticide.

12. Use according to claim 11 against
mosquitoes, preferably disease vector mosquitoes,
more preferably *Aedes* spp. (*Aedes aegypti, Aedes japonicus, Aedes albopictus*), *Culex* spp. and *Culex pipiens* spp. (*C*. *nigripalpus, C. pipiens, C. tarsalis, C. annulirostris, C. univittatus, C. quinquefasciatus*), *Anopheles* spp. (*Anopheles quadrimaculatus, Anopheles gambiae, Anopheles stephensi, Anopheles maculipennis, Anopheles funestus, Anopheles arabiensis*),
*Stegomyia aegypti, Stegomyia albopicta, Aedimorphus vexans,*
wherein the use is preferably directed against the larvae of said mosquitoes (larviciding),
and/or wherein the use is in combination with further insecticide(s) against mosquito larvae, such as
methionine
temephos
*Bacillus thuringiensis* subspecies *israelensis* toxins (*Bti* toxins),
*Lysinibacillus sphaericus* powder SPH88,
chitin synthesis inhibitor(s), e.g. diflubenzuron, novaluron, pyriproxyfen,
methoprene,
anethole,
cinnamaldehyde,
cinnamyl acetate,
or combinations thereof.

13. Use according to claim 11 against insect pests attacking crop plants and/or ornamental plants,
such as
- *Lepidoptera,*
such as *Ostrinia nubilalis* (European corn borer), *Spodoptera exigua, S. frugiperda, S. littoralis, Pectinophora gossypiella, Heliothis virescens, Helicovera zea, Chrysodeixis includes, Helicoverpa armigera,* cutworms *(*various species including *Agrotis* spp., *Peridroma saucia, Nephelodes minians),* hornworms (*Manduca quinquemaculata, Manduca sexta*), Lepidoptera complex (e.g. rice stem borers *Chilo auricilius, C. suppressalis, Scirpophaga incertulas, S. innotata*)*, Grapholita funebrana, Phthorimaea operculella, Elasmopalpus lignosellus*),
- *Coleoptera,*
such as Colorado potato beetle (*Leptinotarsa decemlineata*)*, Diabrotica spp.*, *Anthonomus grandis* (boll weevil), *Oulema melanopus, O. gallaeciana*
- sucking insects
such as Thrips (Thysanoptera, e.g. *Thrips tabaci, Frankliniella schultzei*), Aphids (Hemiptera, e.g. *Aphis gossypii, Dysdercus koenigii, Creontiades dilutes*), white flies (Homoptera),
- Arachnida
such as *Tetranychus urticae, Panonychus ulmi* (European red mite),
wherein the use is preferably in combination with further insecticide(s), such as
*Bacillus thuringiensis* toxins (*Bt* toxins),
azadirachtin,
chitin synthesis inhibitor(s), e.g. diflubenzuron,
tebufenozide,
methoprene,
malathion,
or combinations thereof.

14. Use according to claim 11
- against mite (Acaridae),
such as against *Brennandania lambi, Tyrophagus putrescentiae* (storage mite), *Panonychus ulmi* (European red mite), *Tetranychus urticae,*
- against fungus gnats and fungus pests,
such as against Sciaridae and Phoridae (such as *Lycoriella ingenua*), *Camptomyia corticalis, C. heterobia,* "flies",
- against storage pests,
such as Bruchids [*Callosobruchus chinensis* (L.) and *Callosobruchus maculatus* (F.)], *Caryedon serratus,* storage insect pests, including the Indian meal moth, *Plodia interpunctella* (Hübner) (Lepidoptera: Pyralidae); *Sitophilus spp.* (Coleoptera: Curculionidae); and their natural enemies [e.g., *Cephalonomia tarsalis* (Ashmead) (Hymenoptera: Bethylidae), and *Anisopteromalus calandrae* (Howard) (Hymenoptera: Pteromalidae)],
- - in hygiene,
such as against cockroaches, ants, termites and bed bugs,
and/or
- in crop protection,
such as against slugs, blossom beetle, rape stem beetle, Aphids, *Drosophila susukii,* raspberry weevil, caterpillars, fungus gnats, European grape vine moth, stem borers, European red mite (*Panonychus ulmi*)
wherein the use can be in combination with further insecticide(s).

15. A (bio)insecticide or (bio)pesticide composition comprising
(a) a protein of claim 1, 2 or 7, a nucleic acid molecule of claim 3 or 4 and/or a host cell of claim 5 or 6, and
(b) excipient(s) and/or carrier.

16. The composition of claim 15, furthermore comprising:
(c) further active agent(s),
such as insecticide(s) or pesticide(s),
such as
methionine
temephos
*Bacillus thuringiensis* subspecies *israelensis* toxins (*Bti* toxins),
*Lysinibacillus sphaericus* powder SPH88,
chitin synthesis inhibitors, e.g. diflubenzuron, novaluron,
pyriproxyfen,
methoprene,
anethole,
cinnamaldehyde,
cinnamyl acetate,
*Bacillus thuringiensis* toxins (*Bt* toxins),
azadirachtin,
tebufenozide,
malathion,
or combinations thereof.

17. The composition of claim 15 or 16, which is formulated as a solution (such as liquids, e.g. liquid concentrate), a powder, granules, or baits,
optionally further comprising spreading and/or sticking agent(s).
